# EUROPEAN PATENT SPECIFICATION

(11) **EP 2 101 748 B1**
(45) Date of publication and mention of the grant of the patent: **30.07.2014**
(21) Application number: 07827450.3
(22) Date of filing: 29.11.2007
(51) Int. Cl.: A61K 31/343, A61P 29/00, A61P 25/00, A61P 25/28, A61P 25/22, A61P 25/24, A61P 43/00

(54) **USE OF INCENSOLE AND DERIVATIVES THEREOF FOR NEUROPROTECTION AND FOR THE TREATMENT OF DEPRESSION AND ANXIETY**
VERWENDUNG VON INCENSOL UND SEINEN DERIVATEN ZUR NEUROPROTEKTION UND BEHANDLUNG VON DEPRESSION UND ANGSTZUSTÄNDEN
UTILISATION D'INCENSOLE ET LEURS DÉRIVÉS POUR LA NEUROPROTECTION ET LE TRAITEMENT DE LA DÉPRESSION ET DE L'ANXIETÉ

(30) Priority: 29.11.2006 US 861441 P; 16.08.2007 US 956276 P
(43) Date of publication of application: 23.09.2009
(73) Proprietor: Yissum Research Development Company of the Hebrew University of Jerusalem, Ltd., Jerusalem 91390 (IL); Ariel-University Research and Development Company Ltd., 40700 Ariel (IL)
(72) Inventor: MOUSSAIEFF, Arik, St Jerusalem (IL); MECHOULAM, Raphael, 92581 Jerusalem (IL); FRIDE, Esther, 44837 Ariel (IL); SHOHAMI, Esther, 90805 Mevasseret Zion (IL); BEN NERIAH, Yinon, 90805 Mevasseret Zion (IL); GALLILY, Ruth, 93706 Jerusalem (IL)
(74) Representative: Fleuchaus, Michael A.
(86) International application number: PCT/IL2007/001477
(87) International publication number: WO 2008/065666

(56) References cited:
- WO-A-02/053138
- BANNO ET AL: "Anti-inflammatory activities of the triterpene acids from the resin of Boswellia carteri" JOURNAL OF ETHNOPHARMACOLOGY, ELSEVIER SCIENTIFIC PUBLISHERS LTD, IE, vol. 107, no. 2, 19 September 2006 (2006-09-19), pages 249-253, XP005613499 ISSN: 0378-8741
- MOUSSAIEFF ARIEH ET AL: "Incensole acetate, a novel anti-inflammatory compound isolated from Boswellia resin, inhibits nuclear factor-kappa B activation" MOLECULAR PHARMACOLOGY, vol. 72, no. 6, December 2007 (2007-12), pages 1657-1664 URL, XP009098362 ISSN: 0026-895X
- MOUSSAIEFF A. ET AL.: "Incensole acetate, an incense component, elicits psychoactivity by activating TRPV3 channels"[Online] 20 September 2007 (2007-09-20), XP002475422 Retrieved from the Internet: URL:http://www.ispp.org.il/Program2007.pdf > [retrieved on 2008-04-07]
- A. MOUSSAIEFF ET AL.: "Is incense a pharmacological tool for spiritual exaltation?" NEURAL PLASTICITY, [Online] 25 November 2007 (2007-11-25), - 27 November 2007 (2007-11-27) XP002475423 Retrieved from the Internet: URL:www.hindawi.com/GetPDF.aspx?doi=10.115 5/2007/30585> [retrieved on 2008-04-07]

## Description

### FIELD OF THE INVENTION

This invention relates to uses of incensole, incensole acetate, their derivatives, and pharmaceutical compositions comprising them, for treating various diseases or conditions.

### BACKGROUND OF THE INVENTION

Boswellia species (*Burseraceae*) are native of Eastern Africa, where their resin ("*frankincense*" "*olibanum*") has been widely used as incense and for various medical purposes. For example these species are known as diuretic agents, for the treatment of vasious diseases such as Bilharzia, stomachache syphilis and Rheumatism (Watt, 1962). Boswellia resin was found to be useful for the treatment of inflammations (Singh & Atal, 1986), as well as several diseases associated with inflammatory conditions such as for example active Crohn's disease and Asthma (Gerhardt et al., 2001; Gupta, 1998). It was previously reported that the anti-inflammatory properties of Boswellia resin may be attributed to the Boswellic acid and its derivatives (Ammon et al., 1993).

The use of Boswellia resin for its psychoactive properties extends beyond the Near East and Europe. In Ayurveda, the Indian medical tradition, Boswellia resin is reported to have a 'strong action on the nervous system'. In Ethiopia, where Boswellia trees are indigenous, it is believed to have a tranquilizing effect.
The isolation of IA (incensole acetate) and its structural elucidation was first described by Corsano and Nicoletti (Corsano & Nicoletti, 1967). In 2006 the isolation and investigation of several constituents including incensole of Boswellia resin as well as their anti-inflammatory potential were published (Banno et. al, 2006, Journal of Ethnopharmacology 107, 249-253). US Patent No. 5064823 discloses pentacyclic triterpenoid compounds such as α boswelic acid and its acetate, which have an inhibitory effect on topoisomerase I and topoisomerase II.

WO 02/053138 discloses the use of incensole and/or furanogermacrens, derivatives, metabolites and analogeous thereof for selective inhibition of neoplastic cells, for example for the treatment, inhibition or prevention of precancerous lesions, tumors, cancer growth or other neoplasias in mammals.

NF-κB (nuclear factor-κB) is a collective name for a group of inducible dimeric transcription factors. NF-κB is found in essentially all cell types and is involved in activation of a large number of genes in response to various stressful situations, e.g. infection and inflammation. The subcellular localization of NF-κB is controlled by a family of inhibitory proteins, IκBs, which bind NF-κB and mask its nuclear localization signal, thus preventing nuclear translocation. Exposure of cells to a variety of extracellular stimuli leads to the rapid phosphorylation, ubiquitination, and ultimately proteolytic degradation of IκB, which frees NF-κB to translocate to the nucleus where it regulates gene transcription (Karin and Ben-Neriah, 2000). IκB phosphorylation, followed by its degradation is considered to be the major step in NF-κB regulation (Ghosh & Karin, 2002).

Traumatic brain injury (TBI) is often associated with permanent cognitive disorders, learning disabilities and various behavioral and emotional problems. Despite promising pre-clinical data, most of the clinical trials conducted so far have failed to demonstrate any significant improvement in outcomes, mainly because of ineffective therapies or because of the selection of inappropriate target mechanisms (Marmarou et al, 2005, Narayan et al, 2002). Secondary brain damage, triggered by the initial impact, develops over hours, weeks and even months following injury. Secondary brain damage can increase mortality and worsen disability but, unlike the primary lesion, may potentially be attenuated by appropriate treatment. TBI induces early phase neuronal activation of NF-κB, followed by its remarkably prolonged activation (Beni et al, 2004) even up to 1 year (Nonaka, 1999). Studies on the role of NF-κB in the brain following closed head injury in (CHI) mice have revealed that inhibition of acute NF-κB activation is associated with enhanced functional recovery (Beni et al, 2004).

### SUMMARY OF THE INVENTION

The invention relates to a compound having the structural formula I, including enantiomers, diastereomers, solvates, and pharmaceutically acceptable salts thereof: 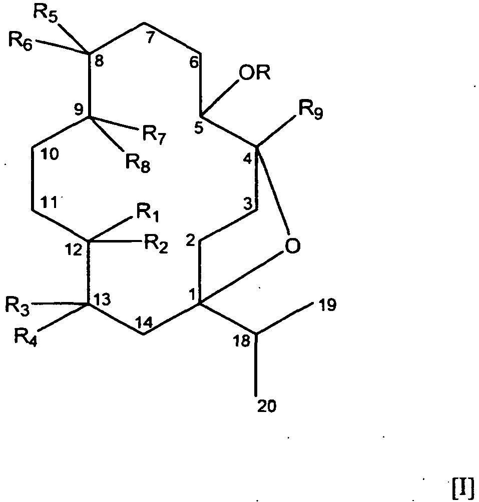 wherein,
**R** is selected from H, -C(=O)R', and -C(=O)OR", wherein R' is C₁₋₂₅alkyl and R" is H or C₁₋₂₅alkyl;
**R₁, R₂, R₅,** and **R₆** are independently selected from H, OH and CH₃;
**R₃, R₄, R₇,** and **R₈** are independently selected H and OH;
**R₉** is H or CH₃; or
one of **R₁** and **R₂** and one of **R₃** and **R₄** taken together form (i) a second bond between C₁₂ and C₁₃ or (ii) an epoxide ring, along with the carbon to which they are bonded; and/or
one of **R₅** and **R₆ and** one of **R₇** and **R₈** taken together form (iii) a second bond between C₈ and C₉ or (iv) an epoxide ring, along with the carbon to which they are bonded; and/or
one of **R₅** and **R₆** together with R form a single bond, thereby forming an epoxide ring along with the carbon to which they are bonded,
for use in neuroprotection. Moreover, the invention relates to a compound having the structural formula I for use in the treatment, prevention or amelioration of a disease or condition selected from depression, anxiety, obsessive compulsive behaviors, deterioration in cognitive function, and deterioration in neurobehavioral function.

The invention further relates to a TRPV3 agonist having the structural formule I for use in treating a disease or condition selected from mood-disorders, anxiety, and a combination thereof.

### BRIEF DESCRIPTION OF THE DRAWINGS

In order to understand the invention and to see how it may be carried out in practice, embodiments will now be described, by way of non-limiting example only, with reference to the accompanying drawings, in which:

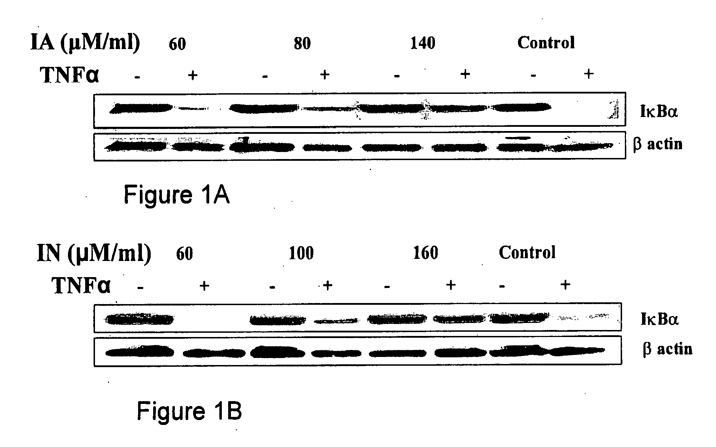

**Figure 1****: Shows that IA (incensole acetate) and IN (incensole) inhibit IκBα degradation in a dose dependant manner.** HeLa cells were pre-incubated with IA (Fig. 1A) or IN (Fig. 1B) at the indicated concentrations for 2 hrs prior to 20 minutes exposure to TNFα (20 ng/ml). At least three more experiments were repeated with highest indicated dose, resulting similarly.

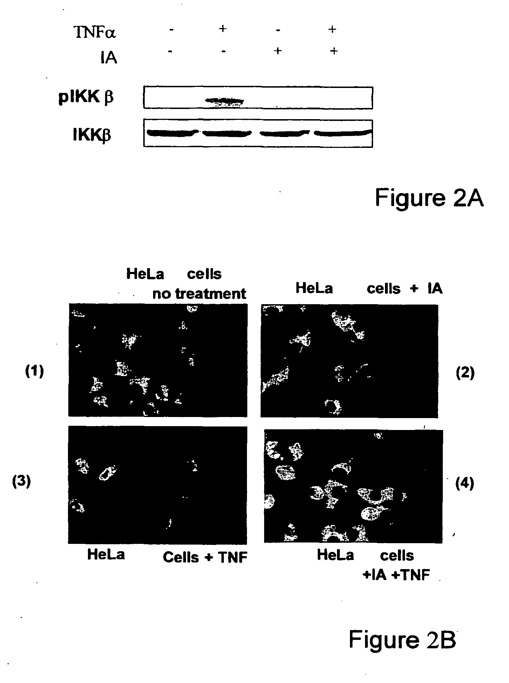

**Figure 2****: Shows that IA impairs IKK phosphorylation upstream of IKK, thus inhibiting IκBα degradation and NF-κB accumulation in cell nuclei. (A)** IA inhibits IKK phosphorylation. HeLa cells were stimulated with TNFα (20 ng/ml for 20 minutes) in the absence or presence of IA (140 µM) as shown. Whole cell extracts were prepared and analyzed for the phosphoryation of IKKα and IKKβ by Western blotting (WB). **(B)** HeLa cells were stimulated with TNFα (20 ng/ml for 20 minutes) in the absence or presence of IA (140 µM). Cells were fixed and then stained with rabbit anti-p65 followed by anti-rabbit Rhodamine Red-labeled secondary Ab, and with DAPI for nuclei location (not shown). (B1) HeLa cells, no treatment; (B2) HeLa cells + IA; (B3) HeLa cells +TNF; (B4) HeLa cells +IA+TNF. The cells were examined under an Axioscope Zeiss microscope with a plan-Neofluor * 60 lens. Results of one of three independent experiments are shown.

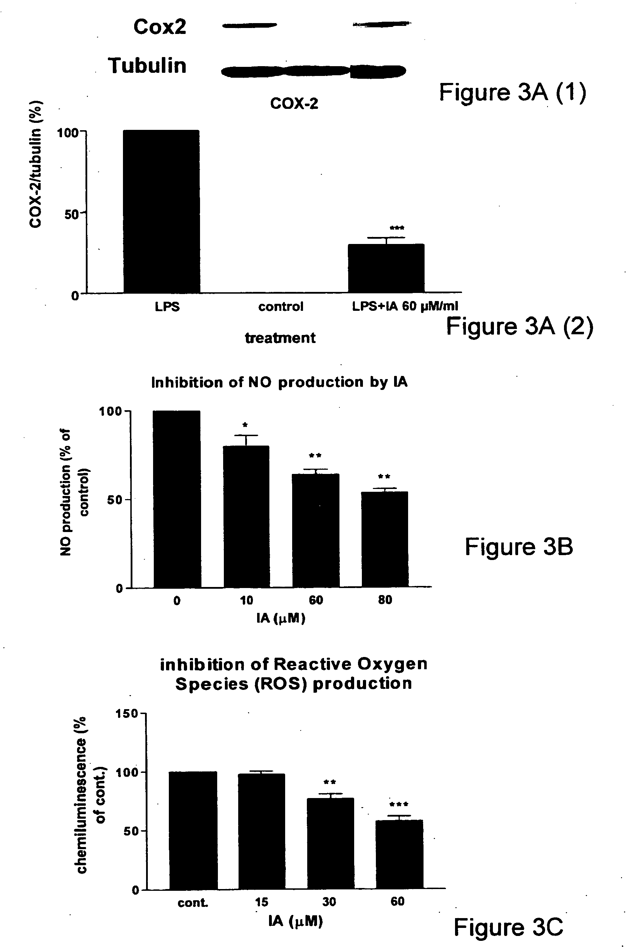

**Figure 3****: Shows IA activity on inflammatory mediators levels. (A(1))** Representative Western Blot bands of Cox2 are shown with tubulin as reference. **(A(2))** COX-2 levels were measured in RAW 264.7 cells incubated for 24 hrs,. with LPS in the presence or in the absence of IA (60 µM/ml); **, p<0.001. **(B)** Murine peritoneal macrophages were activated by LPS (1 µg/ml for 24 hrs.) in absence of IA or in the presence of IA at indicated concentrations. NO generation was determined by measuring the nitrite accumulated in the supernatants; **, p<0.001. **(C)** Generation of ROS by RAW 264.7 macrophages (5 x 10⁵ cells in 0.5 ml Hanks' balanced salt solution) was measured by chemiluminescence. Cells were pre-incubated with various doses of IA for 24 hrs before luminol (10 µl) and zymosan (30 µl) were added to the tubes; *, p<0.05; **, p<0.01; ***, p<0.001

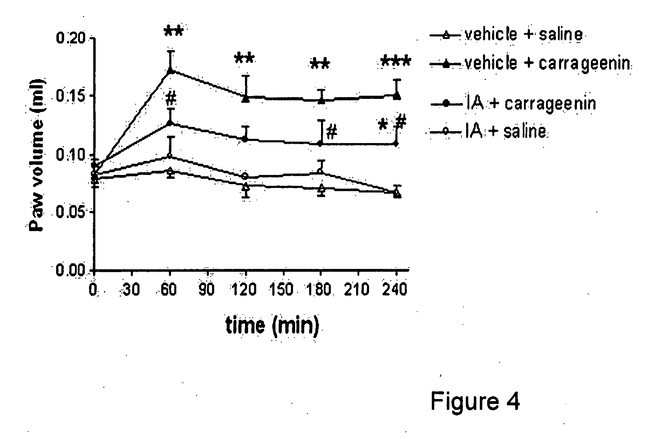

**Figure 4****: Shows that IA inhibited inflammation in the inflamed paw model after injection of carrageenin.** IA (50 mg/kg) or vehicle were injected i.p. to Sabra female mice (5 per group) 30 min before induction of the inflammatory stimulus. Hind paws were then injected with 50 µl of saline or λ-carrageenin (4%). Ensuing inflammatory swelling was measured by increase in foot volume in a plethysmometer. IA also reduced paw redness (as a measure of erythema) and licking (as a measure of pain) (data not shown). There were highly significant effects of treatment (F=11.7, df=3,64, P<0.001). *, different from IA + saline, P<0.05; **, ***, different from vehicle + saline at P<0.01, P<0.001 respectively; #, different from vehicle + carrageenin, P< 0.05.

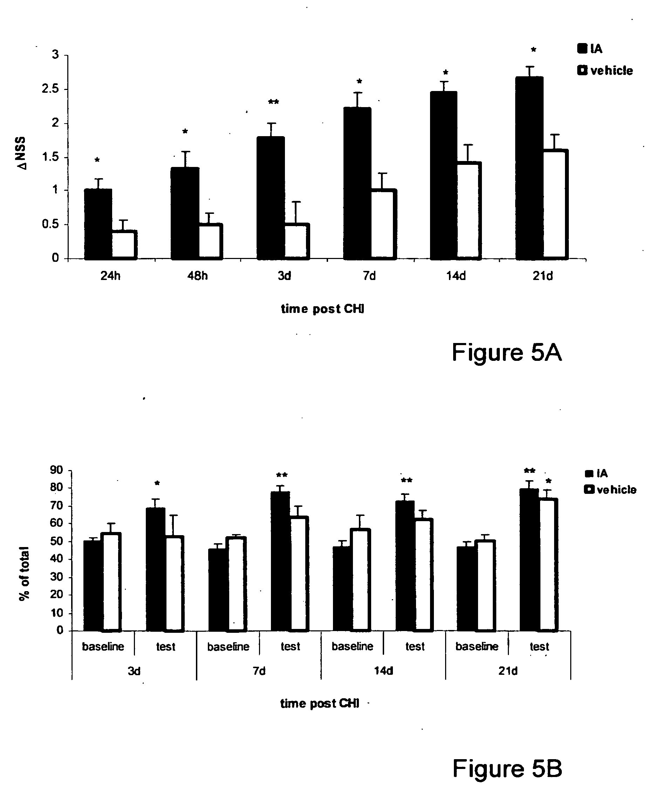

**Figure 5****: Shows the beneficial effect of IA (50 mg/kg) on neurobehavioural recovery and cognitive function following closed head injury (CHI). (A)** Motor function was assessed at 1h after CHI and up to 21 days and is expressed as ΔNSS (Example 5). ΔNSS values were significantly higher in IA-treated (filled bars) as compared to vehicle treated (empty bars) mice. This effect was sustained from 24h to 21 days following injury as determined by the Mann-Whitney test (n =9-10 per group; * p<0.01; **pH0.001, as compared to vehicle treated, at the same day). **(B)** Mice were subjected to the object recognition test (Example 6) 3, 7, 14 and 21 days after CHI. The absolute time spent exploring each of the two objects was recorded and the % time calculated. At baseline (bl), when presented with two identical objects, exploration time of each object was about 50% in both groups. In the test (T) situation, after one of the objects was replaced by a novel one the % time spent exploring the new object was calculated. IA mice spent a significantly higher percentage of their exploration time near the novel object (*p≤ 0.01; **p<0.001 as compared to baseline measurement at the same day) whereas the vehicle-treated mice demonstrated a severe deficit on this test and could not distinguish between the two objects (n=3-5/group).

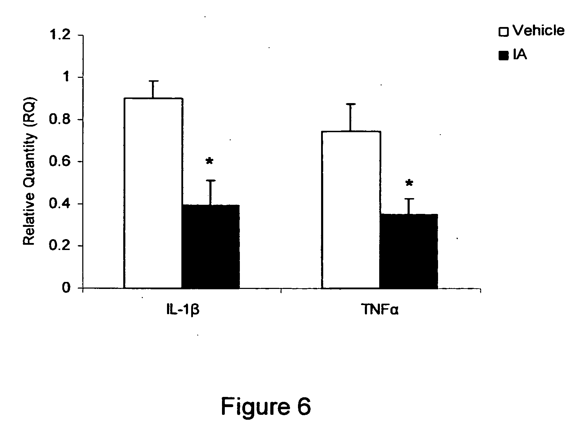

**Figure 6****: Shows that IA (50 mg/kg) inhibits IL-1β and TNFα mRNA expression following closed head injury.** IL-1β and TNFα mRNA levels were quantified 3 hours post-injury by real time polymarerase chain reaction. β-actin was used as endogenous control. *p<0.05 vs. vehicle, as determined by student's t-tests.

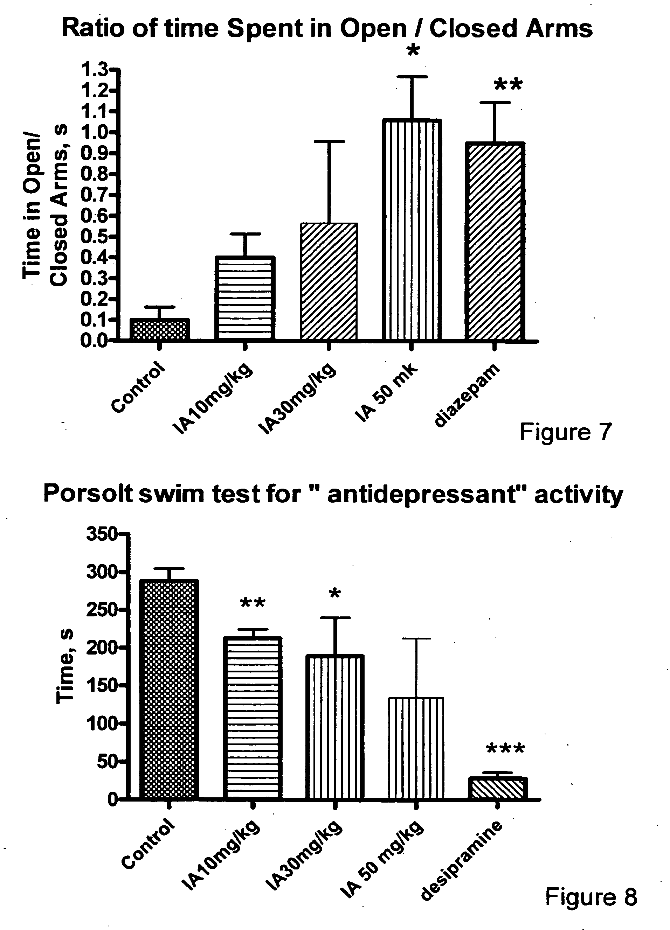

**Figure 7****: Shows that IA exerts a potent and dose dependent effect in the plus-maze test, indicating an anxiolytic effect.** Mice (female Sabra strain, aged 3.5-4.5 months old) were injected intraperitoneally with 10, 30 or 50 mg/kg of incensole acetate or with vehicle. Each dose was administered to 5 mice. Fourty five min after injection the mice were tested in the plus-maze for 'anti-anxiety' effects. Diazepam (5 mg/kg) was injected to a separatae group of mice as a positive control. One-way Anova indicated significant effects (F = 4.2, df=4,32, P<0.01). Data are presented as means ± SEM. *, P<0.05; **, P<0.01 compared to vehicle.
**Figure 8****: Shows that IA exerts a potent and dose dependent anti-depressive effect in the Porsolt forced swimming test, indicating an anti-depressant effect.** Mice (female Sabra strain, aged 3.5-4.5 months old) were injected intraperitoneally with 10, 30 or 50 mg/kg of incensole acetate or with vehicle. Each dose was administered to 5 mice. Fifty min after injection the mice were tested in the Porsolt forced swimming test for 'anti-depressant' effects. Desipramine (5 mg/kg) was injected to a separate group of mice as a positive control. One-way Anova indicated significant effects (F = 8.9, df=4,27, P<0.01). Data are presented as means ± SEM. DMI=desipramine. *, P<0.05; **, P<0.01; ***, P<0.001 compared to vehicle.

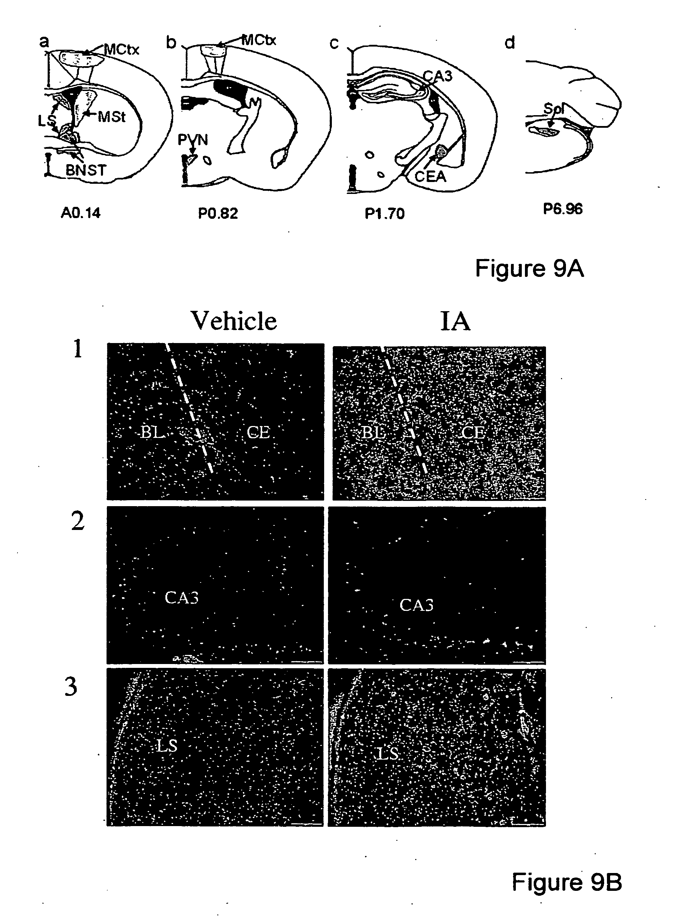

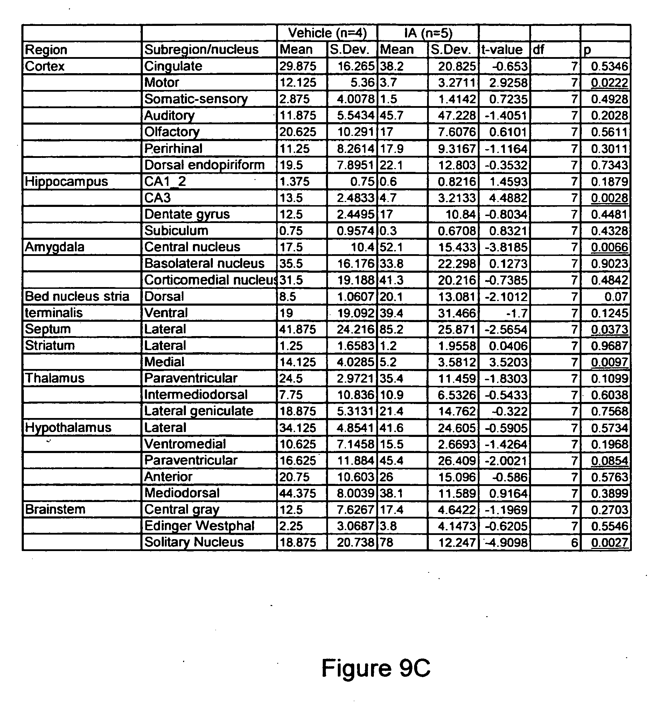

**Figure 9****: Shows that IA modulates c-Fos expression in several brain areas.** The diagram **(****Fig. 9A****)** illustrates brain areas of female Sabra mice (15-20 weeks; n = 4-5) where IA (50 mg/kg) significantly changed the number of c-Fos-immunoreactive cells, 60 min after i.p. injection of IA or vehicle. The drawings were modified from plates 30, 38, 45, 89 respectively from Paxinos and Franklin (2001). The atlas sections are arranged from anterior a to posterior d. The number under each section indicates its distance (mm) from the bregma. "A" is anterior to bregma and "P" is posterior to bregma. IA significantly increased c-Fos in the lateral septum (LS), central nucleus of the amygdala (CEA) and solitary complex (Sol). IA significantly reduced c-Fos in the motor cortex (MCtx), medial striatum (MSt) and hippocampal CA3 region (CA3). **Fig. 9B** shows representative micrographs and **Fig. 9C** (Table 1) quantification.

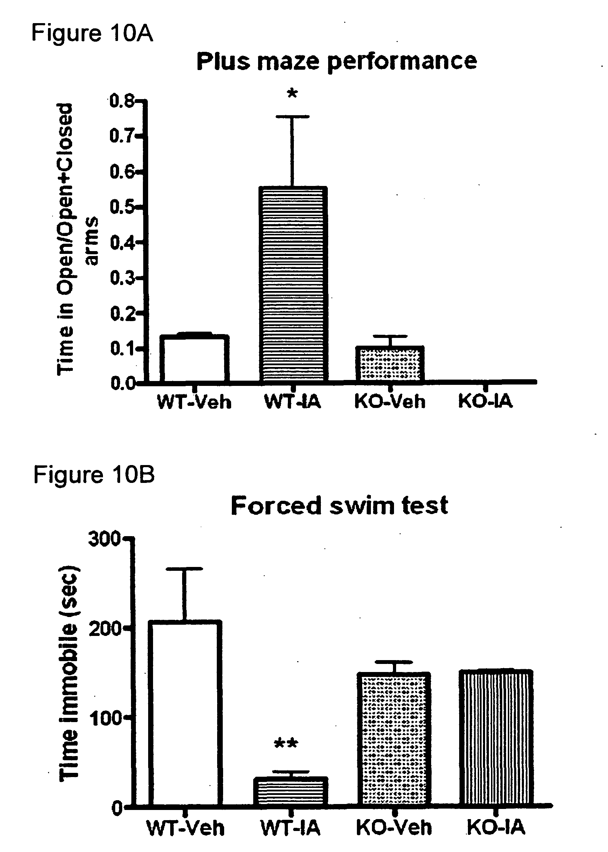

**Figure 10****: Shows that IA exhibits an anti-depressant-like effect in the Porsolt forced-swimming test and an anxiolytic effect in the elevated plus maze in WT, but not TRPV3^{-/-} mice.** Wild type and TRPV3^{-/-} mice (18-20 weeks old) were injected with vehicle (isopropanol:emulphor:saline =1:1:18) or IA (75 mg/kg). 30 min
**Figure 10****: Shows that IA exhibits an anti-depressant-like effect in the Porsolt forced-swimming test and an anxiolytic effect in the elevated plus maze in WT, but not TRPV3^{-/-} mice.** Wild type and TRPV3^{-/-} mice (18-20 weeks old) were injected with vehicle (isopropanol:emulphor:saline =1:1:18) or IA (75 mg/kg). 30 min later they were tested in **a,** the elevated plus maze (5 min), followed by **b,** 9 min exposure to the Porsolt forced-swimming test. In the elevated plus maze assay, IA caused wild type (WT) mice to spend significantly more time in the aversive open arms of the maze (relative to the total time spent in both arms). In the Porsolt forced-swim test, immobility was significantly reduced by IA in WT mice, whereas TRPV3 knockout (KO) mice did not respond to IA. No difference was noted in WT and TRPV3 KO mice in response to vehicle. Data are presented as means ± SEM; *n* = 4-5. * *p* < 0.05, compared to WT-Vehicle-injected mice (Bonferroni post hoc test). ** *p* < 0.01, compared to WT-Vehicle-injected mice (Bonferroni post hoc test).

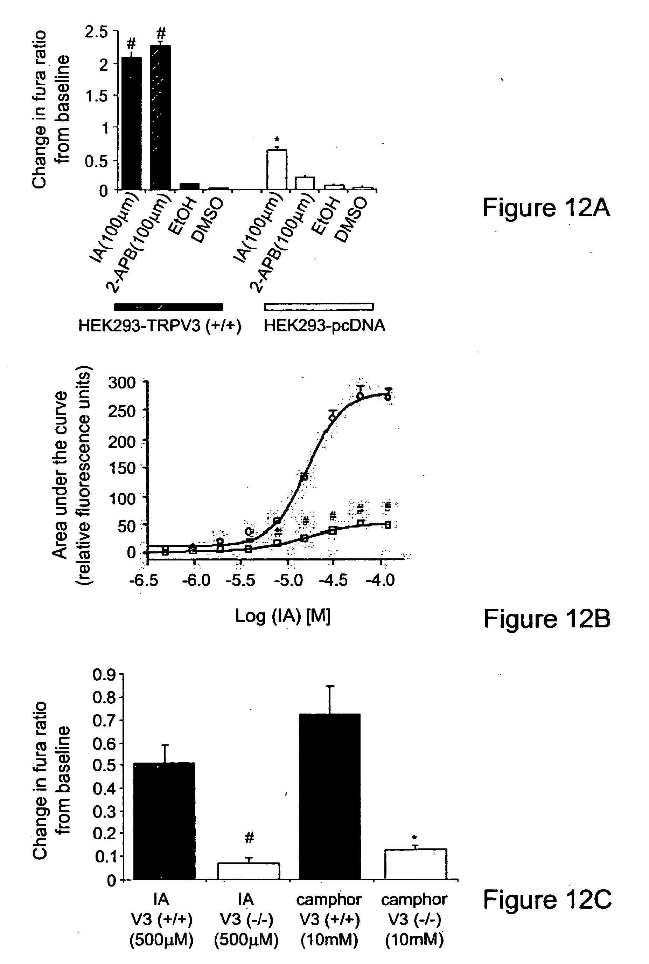

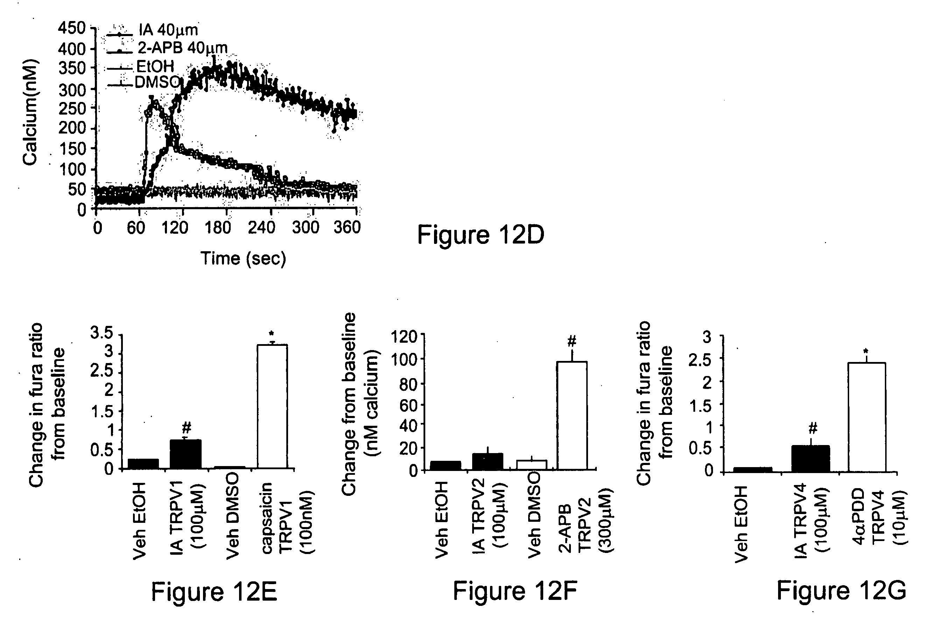

**Figure 12****: Shows that IA is a potent TRPV3 activator (agonist). a,** IA or 2-APB evoked robust calcium increases in mouse HEK293 TRPV3-YFP transfected cells compared with vehicle, #, *p* < 0.001 *(n* = 9). IA treated HEK293-TRPV3(+) cells show a significantly higher activation than HEK293-pcDNA cells, * *p* < 0.001 (n = 9). **b**, IA dose-dependently induced calcium influx in TRPV3-YFP transfected HEK293 cells in the presence of calcium in the extracellular media , ○, EC₅₀ = 16 µM, Hill slope = 2.2, *(n* = 10). In the absence of calcium, □, the effect of IA was markedly reduced, #, *p* < 0.05 (n = 5). **c**, IA (500 µM) increased intracellular calcium levels in primary keratinocytes from TRPV3^{+/+} but not TRPV3^{-/-} mice. Camphor (10 mM) showed a similar effect. *; #, *p* < 0.005 (n = 6), *t* test (two-tailed). **d**, Representative single cell robustly increased calcium in these cells. #, *p* < 0.001 *(n* = 41-51). **g**, IA induced a very modest calcium influx in rat TRPV4 transfected HEK293 compared to vehicle, #, *p* < 0.001 (n = 26). 4αPDD induced a robust calcium increase that was significantly larger than the effect of IA, *, *p* < 0.001 *(n* = 26). All error bars indicate SEM; *p* values in all subfigures but c represent analysis with one-way ANOVA Bonferroni's post hoc.

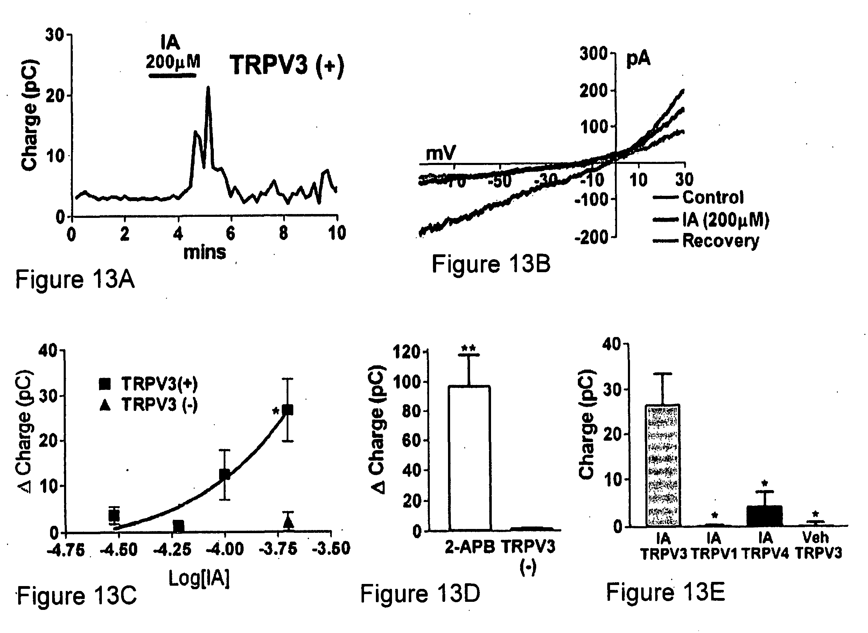

**Figure 13****: Shows that IA activates a TRPV3 current when it is stably expressed in HEK293 cells.** a, Sample time course shows summed charge of current activated (-85 to -45 mV, in pC) with application of IA (200 µM). **b**, Sample current response to voltage ramp from same cell as a. c, Dose response for IA shows activation of currents at 200 µM in TRPV3(+) HEK293 cells (■), but not in TRPV3(-) controls (▲); *, *p* < 0.001 1-way ANOVA with Dunnett's posthoc vs. TRPV3(-). **d**, TRPV agonist 2-APB (100 µM) activates currents in TRPV3(+) cells but not in TRPV3(-) cells; **, *p* < 0.001, unpaired two-tailed Student's t-test. **e**, IA (200 µM) does not activate currents in TRPV1(+), TRPV4(+) cells, nor does vehicle in TRPV3(+) cells. TRPV3(+) response to IA is shown for reference. *, *p* < 0:001. Error bars represent SEM, n = 4-5.

### DETAILED DESCRIPTION OF THE INVENTION

The present invention is based on the finding that incensole (IN) and incensole acetate (IA), possess various pharmacological activities which were not previously attributed to the isolated compounds per-se.

In the first aspect of the invention, there is provided a compound having the structural formula I, including enantiomers, diastereomers, solvates, and pharmaceutically acceptable salts thereof: 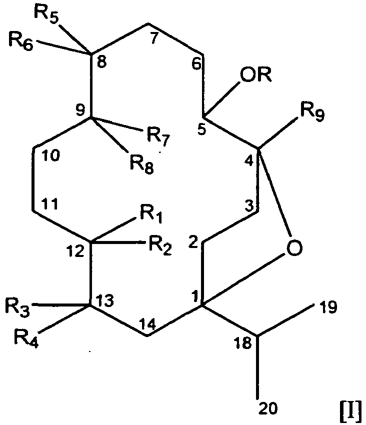 wherein,
**R** is selected from H, -C(=O)R', and -C(=O)OR", wherein R' is C₁₋₂₅alkyl and R" is H or C₁₋₂₅alkyl;
**R₁, R₂, R₅,** and **R₆** are independently selected from H, OH and CH₃;
**R₃, R₄, R₇,** and **R₈** are independently selected H and OH;
**R₉** is H or CH₃; or
one of **R₁** and **R₂** and one of **R₃** and **R₄** taken together form (i) a second bond between C₁₂ and C₁₃ or (ii) an epoxide ring, along with the carbon to which they are bonded; and/or
one of **R₅** and **R₆** and one of **R₇** and **R₈** taken together form (iii) a second bond between C₈ and C₉ or (iv) an epoxide ring, along with the carbon to which they are bonded; and/or
one of **R₅** and **R₆ together** with R form a single bond, thereby forming an epoxide ring along with the carbon to which they are bonded,
for use in neuroprotection.

By the term *"one of **R**₁ and **R₂** and one of **R₃** and **R₄** taken together form a second bond between C₁₂ and C₁₃*" is meant that the bond formed between *C₁₂ and C₁₃* is a π bond, thereby the bond between C₁₂ and C₁₃ is a double bond.

Similarly by the term *"one of **R₅** and R₆ and one of **R₇** and **R₈** taken together form (iii) a second bond between C₈ and C₉"* is meant that the bond formed between C₈ *and* C₉ is a π bond, thereby the bond between C₈ and C₉ is a double bond.

It is appreciated that double bond conformations are also within the scope of the present invention.

As used herein the term ***"C₁₋₂₅ alkyl"*** refers to a saturated aliphatic hydrocarbon of 1 to 25 carbon atoms. The C₁₋₂₅ alkyl may be a straight or a branched alkyl.

Whenever a numerical range e.g. "1-25" is stated herein, it means that the group in this case the alkyl group, may contain 1 carbon atom, 2 carbon atoms, 3 carbon atoms, etc., up to and including 25 carbon atoms.

According to one embodiment of the present invention, the compound is incensole or incensole acetate. The structures of these compounds are shown below.

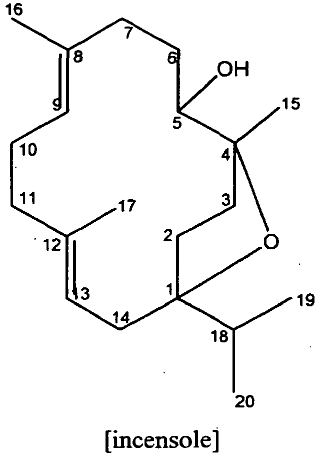

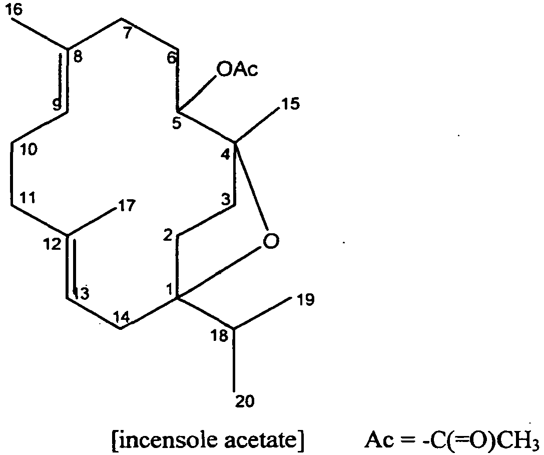

The term ***"treatment, prevention or amelioration"*** in connection with the inflammatory disease aspect concerns improvement of at least one undesired manifestation of the disease such as: increase in disease free periods, decrease in acute disease periods (in time and severely), decrease in severity of the disease , improvement in life quality, decreased mortality as well as prophylactic treatment before disease occurs.

As used herein the term **"*medicament*"** refers to a pharmaceutical composition. Specifically, it refers to a pharmaceutical composition comprising at least one compound of structural formula I described in the present invention in any suitable pharmaceutical acceptable carrier (e.g. an excipient or diluent), and also to different formulations required for different routes of administration. For example the medicament may be formulated for oral administration, or may be formulated for parenteral, rectal or other modes of administration.

The active ingredients of a pharmaceutical composition as disclosed herein may include at least one compound of formula I, i.e. a single compound, or two or more compounds.

By the term **"*consisting essentially of*"** in connection with a pharmaceutical composition is meant that the active ingredient includes one or more compounds of formula I as defined above and is substantially free of other active compounds. By the term **"*substantially free of other active compounds*"** is meant that the active ingredient includes at least 70%w/w of a compound of formula I, more preferably at least 80%w/w, more preferably at least 90%w/w, even more preferably at least 95% w/w of a compound of formula I. The active ingredient may include at least one of the above indicated concentrations of compound of formula I and up to 99.9% w/w of compound of formula I. The active ingredient may also include at least one of the above indicated concentrations and up to 99%w/w of compound of formula I.

By yet a further aspect of the invention there is provided a compound having the structural formula I as hereinabove defined for use in neuroprotection.

In one embodiment said neuroprotection is for treatment, prevention or amelioration of a disease or condition resulting from injury, trauma, or CNS neurodegenerative diseases.

The term **"*treatment, prevention or amelioration*"** in connection with neuroprotection as used herein, means treating, preventing, or reversing cognitive decline associated with concentration loss, memory-acquisition loss, and information-storage or retrieval loss including, but not limited to, neuronal disorders, such as cognitive decline associated with aging, cognitive impairment and neurodegenerative disorders, such as Alzheimer's disease, Parkinson's disease, ALS, Huntington Chorea, HIV associated dementia, Lewy body dementia, multiple sclerosis, and prion disease. The term also includes treating, preventing, or reversing neuronal dysfunction associated with loss of motor skills (ataxia), such as Parkinson's disease and amyotrophic lateral sclerosis as well as neuronal dysfunction resulting from CNS injury, such as head trauma, stroke, spinal-cord injury, and peripheral-nerve injury.

As used herein the term **"*neurodegenerative disease*"** refers broadly to disorders or diseases that affect the nervous system and are characterized by gradual neuronal loss and/or gradual loss of neuronal function, including but are not limited to age-associated memory impairment, Parkinson's disease, Alzheimer's disease, Huntington's chorea disease, multiple sclerosis and amyotrophic lateral sclerosis (ALS), HIV associated dementia, Lewy body dementia, and prion disease.

In another one of its aspects the present invention provides a compound having the structural formula I for use in the treatment, prevention or amelioration of a disease or condition selected from depression, anxiety, obsessive compulsive behaviors, deterioration in cognitive function, deterioration in neurobehavioral function, and combination of any of the above.

The term **"*deterioration of cognitive and*/*or neurobehavioral function*"** refers to decrease in learning and memory capacitates, to decrease in orientation in time and space and decrease in coordination, and movement capacities due to CNS function. The deterioration may be a natural result of aging but may also be as a result of injury, trauma (caused by accidents, stroke, surgery or diseases) or of disease in the CNS notably neurodegenerative diseases.

The terms **"*injury*"** and **"*trauma*"** includes physical injury to the CNS (or head) as a result of physical insult, injury or damage due to stroke, ischemia, hypoxia, surgery or a disease such as an infectious disease in the CNS (such as AIDS -associated dementia) as well as a neurodegenerative disease, for example Alzheimers, Parkinson, Hungtinton Chorea or old age dementia.

The term **"*treatment, prevention or amelioration of depression, anxiety or obsessive compulsive behavior"*** refers to decrease or elimination of the severity of the condition, decrease in the duration of the episode as well as preventive treatment in individuals prone for such conditions to avoid or minimize the entry to these undesired episodes. The term **"*treatment*"** in connection with depression concerns improvement of at least one undesired manifestation of the disease such as anorexia and bulimia as well as the manifestation of clinical depression.

It is demonstrated in the present invention that incensole acetate (IA), a *Boswellia* resin constituent, is a potent TRPV3 agonist that causes anxiolytic-like and antidepressive-like behavioral effects in wild type (WT) mice with concomitant changes in c-Fos activation in the brain. These behavioral effects were not noted in TRPV3^{-/-} mice, suggesting that they are mediated via TRPV3 channels. IA robustly activated TRPV3 channels stably expressed in HEK293 cells and in keratinocytes from TRPV3^{+/+} mice. It had no effect on keratinocytes from TRPV3^{-/-} mice and showed modest or no effects on TRPV1, TRPV2 and TRPV4. The results shown below (see Example 15) imply that TRPV3 channels in the brain play a role in emotional regulation.

In a further aspect of the invention, there is provided a TRPV3 agonist having the structural formula I for use in treating a disease or condition selected from mood-disorders, anxiety, and a combination thereof.

As used herein the term **"*mood disorders*"** refers to an emotional and/or behavioral disturbance characterized by persistent and pervasive bouts of euphoria and/or depression. Exemplary mood disorders include depression and bipolar disorders (also known as manic depressive illness). Anxiety is frequently associated with mood disorders, such as depression.

By a specific embodiment the mood-disorder is depression.

Said TRPV3 agonist is a compound having the structural formula I as defined hereinabove. In yet a further embodiment said compound is incensole or incensole acetate.

The invention relates to a compound having structural formula I for use in the treatment, prevention, or amelioration of a disease or condition selected mood disorders, anxiety, and a combination thereof.

### Compounds of the Invention:

In one embodiment R' and/or R" of structural formula I are each independently C₁₋₂₀alkyl; in a further embodiment C₁₋₁₅alkyl; in yet a further embodiment C₁₋₁₀alkyl; in a further embodiment C₁₋₆alkyl; in an additional embodiment C₁₋₅alkyl.

In another embodiment, the bond between carbons 8,9 and\or 12,13 is a single bond. In a further embodiment carbons 8,9 and\or 12,13 form an epoxide ring, along with the carbon to which they are bonded. In yet a further embodiment, the substituents on carbons 8,9 and\or 12,13 are substituted as to form a diol. In another embodiment one or more of R₁, R₂, R₅, R₆, and R₉ is H.

In one embodiment of the present invention, the compound is incensole or incensole acetate.

Compounds used by the methods and uses of the invention may be synthesized by the synthetic routes described and detailed in G. STRAPPAGHETTI, G. PROIETTI, S. CORSANO, AND I. GRGURINA. Synthesis of incensole. BIOORGANIC CHEMISTRY 11, 1-3 (1982) and T. Kato, C.C. Yen, T. Kobayashi, Y. Kitahara. Cyclization of polyenes XXI. Synthesis of DL-incensole. Chemistry letters 1191-1192 (1976). The derivatives of structural formula I may be synthesized by procedures as described in FessendenR.J. & Fessenden J.S.; Organic chemistry, 1990, Brooks/Cole Publishing company, California (pp. 257-301 (alcohols), 301-323 (ethers and epoxides), 529-591 (aldehydes and ketones), 591-627 (Derivatives of carboxylic acids), 391-448 (double bonds)). Synthesis procedures can be also found in additional general textbooks, for example, Morrison R.T. & Boyd R.N.; Organic chemistry, 1992, Pramount communication company, California.

### Pharmaceutical compositions, dosages, and routes of administration

As used herein a **"*pharmaceutical composition*"** refers to a preparation of one or more compounds described herein, with other inert chemical components such as suitable pharmaceutically acceptable carriers. The purpose of a pharmaceutical composition is to facilitate administration of a compound to a mammal.
As used herein the term **"*pharmaceutically acceptable carrier*"** refers to an inert nontoxic carrier or diluent that does not cause significant irritation to a subject (mammal) and does not abrogate the biological activity and properties of the administered compound.

Examples without limitation of carriers are lactose, sucrose, water, organic solvents, and polyethyleneglycol.

The carriers may include additional excipients such as binders, disintegrants, lubricants, surface active agents, preservatives and favoring agents.
According to one embodiment of the present invention the route of administration of the composition is selected from oral, parenteral, inhalation, topical, transdermal, nasal, trnsmucosal (e.g. intranasal), intestinal, and rectal.

Additionally according to a preferred embodiment of the present invention the parenteral route of administration is selected from intravenous, intramuscular, intraperitoneal and subcutaneous administration.

Additional suitable routes may be for example intramedullary, intrathecal, direct intraventicular, and intraocular injections.

A specific embodiment is the oral route of administration.

The pharmaceutical composition may be formulated as to provide immediate release or sustained release of the active ingredient from the dosage form after administration to a patient by employing procedures well known in the art.

The final form of the composition includes but not limited to a liquid, a syrup, an elixir, an emulsion, a suspension, drops, a spray, a cream, an ointment, a lotion, a gel, a paste, a powder, a granule, a tablet, a caplet, a pill, a capsule, a suppository, a transdermal patch or an injection.

The pharmaceutically acceptable carrier selected for preparing the pharmaceutical compositions of the present invention depends on the final form of the composition.

Typically, such carriers include additional excipients such as binders, disintegrants, adsorbents, lubricants, wetting agents, buffering agents and surface active agents.

The pharmaceutical compositions are preferably present in a unit dosage form. Unit dosage form as used herein refers to physically discrete units suited as unitary dosages for the mammalian subject to be treated, such as a tablet, a capsule, or powders in vials or ampoules, each unit containing a predetermined quantity of the active ingredient calculated to produce the desired therapeutic effect.

Preferably the pharmaceutical composition in a unit dosage form comprises a therapeutically effective amount of the active ingredient in an amount from 0.1 mg to 1000 mg, more preferably 1 to 500 mg.

Oral dosage forms suitable for oral administration may be presented as discrete pharmaceutical unit dosage forms, such as capsules, cachets, soft elastic gelatin capsules, tablets, caplets, or aerosols sprays, each containing a predetermined amount of the active ingredients, as a powder or granules, or as a solution or a suspension in an aqueous liquid, a non-aqueous liquid, an oil-in-water emulsion, or a water-in-oil liquid emulsion. Dosage forms such as oil-in-water emulsions typically comprise surfactants such as an anionic surfactant, for example anionic phosphate ester or lauryl sulfates, but other types of surfactants such as cationic or nonionic surfactants may be used in the compositions of the present invention. See generally, Remington's Pharmaceutical Sciences, Mack Publishing, Easton Pa., latest edition.

For the purpose of preparing a tablet dosage form, various pharmaceutical carriers which are well-known in this field can be widely used. As to the examples of carriers, excipients such as lactose, sodium chloride, glucose, starch, calcium carbonate, kaolin, cellulose, aluminum silicate and the like may be used; the binders may be for example water, ethanol, propanol, glucose solution, starch solution, gelatin solution, carboxymethyl cellulose, shellac, methyl cellulose, polyvinylpyrrolidone and the like; the disintegrants may be for example starch, sodium alginate, sodium laurylsulfate, sodium starch glycolate and the like; the wetting agents may be for example glycerin, surfactants and the like; the adsorbents may be for example starch, lactose, kaolin, bentonite, colloidal silicic acid and the like; lubricants such as talc, strearates, polyethylene glycols and the like can be used. The tablets preparations can be further shaped into tablets coated with usual tablet coating, for example sugar coated tablets, gelatin film coated tablets, tablets coated with enteric coating, tablets coated with film coating, or double layer tablets and multiple layer tablets.

For the purpose of preparing a capsule dosage form, the compounds of formula [I] as the active ingredients are mixed with the above-mentioned various carriers and the mixture or granules prepared from the mixtures are placed into rigid gelatin capsules or soft capsules.

For the purpose of preparing a suppository dosage form, various carriers which are well-known in this field can be widely used. As to the examples of carries, polyethylene glycols, cacao butter, higher alcohols, esters of higher alcohols, gelatin, semi-synthesized glycerides and the like can be mentioned.

For the purpose of preparing an injection dosage form, liquid preparations, emulsion preparations and suspension preparations are sterilized, further these preparations are preferably isotonic to the blood, and all the diluents which are conventionally used in this field can also be used for example, water, ethyl alcohol, macrogols, propylene glycol, ethyoxylated isostearyl alcohol, polyoxylated isostearyl alcohol and polyoxyethylenesorbitan fatty acid esters.

Additionally, for the purpose of preparing an isotonic injection solutions, an adequate amount of sodium chloride, glucose or glycerin may be added to the injection preparations, further, usual dissolving additives, buffering agents, preservatives and the like may be added.

An example of a pharmaceutical carrier for preparing an injection emulsion preparation is triglyceride emulsion. An example of an acceptable triglyceride emulsion useful in the intravenous and intraperitoneal administration of the compounds of the present invention is the triglyceride emulsion commercially distributed under the tradename Intralipid.RTM.

Moreover, if necessary, coloring agents, preservatives, spices, flavors, sweetening agents and others may be added to the pharmaceutical preparations of the present invention.

Topical preparations such as creams, ointments, pastes, gels, lotions, transdermal patches, inhalants, sprays, aerosols and the like are formulated by using carriers and exipients which are well known in the field.

Methods of preparing the compositions include the step of bringing into association a compound of the present invention with the pharmaceutical carrier. In general, the compositions are prepared by uniformly and intimately bringing into association a compound of the present invention with liquid, semi-solid or solid carriers, and then, if necessary, shaping the product.

The pharmaceutical compositions may be prepared by methods of pharmacy well known to those skilled in the art, e.g. by means of conventional mixing, dissolving, pulverizing, granulating, compressing, emulsifying, levigating, or lyophilizing processes. Techniques for formulation and administration of drugs may be found in "Remington's Pharmaceutical Sciences," Mack Publishing Co., Easton, PA, latest edition.

Pharmaceutical compositions for use in accordance with the present invention may thus be formulated in conventional manner using one or more pharmaceutically acceptable carriers comprising excipients and auxiliaries, which facilitate processing of the active compounds into preparations which, can be used pharmaceutically. The proper formulation is dependent upon the route of administration chosen.

The amount of the active ingredient that may be combined with the pharmaceutical carrier to produce a single dosage form will vary depending upon the mammal treated and the particular mode of administration. For example, a composition intended for oral administration to humans may vary from about 5% to about 95% w/w of the total composition.

Dosage unit forms will generally contain between 0.1 to 1000 mg of the active ingredient, more preferably 1 to 500 mg.

The therapeutically or prophylactically effective amount of an active ingredient administered orally may range from 0.1 to 1000 mg daily, more preferably from 1 to 500 mg daily, either singly or in multiple dosage over 24-hour period. For oral administration, the therapeutically effective amount of the active ingredient may be several times greater than that for parenteral administration.

The above dosages refer to humans.

The desired dose is suitably administered once daily, or several sub-doses, e.g. 2 to 4 sub-doses, are administered at appropriate intervals through the day, or other appropriate schedule.

In the practice of the invention the amount of the compound incorporated in the pharmaceutical composition may vary widely. Factors considered when determining the precise amount are well known to those skilled in the art. Examples of such factors include, but are not limited to, age, sex and weight of the subject being treated, intended medical use of the compounds, severity of the disease, the dosage form, route of administration being employed and the frequency with which the composition is to be administered.

The exact dose may be determined, in accordance with the standard practice in the medical arts of "dose titrating" the recipient; that is, initially administering a low dose of the compound, and gradually increasing the dose until the desired therapeutic effect is observed.

The ratio between toxicity and therapeutic effect for a particular compound is its therapeutic index and can be expressed as the ratio between LD50 (the amount of compound lethal in 50% of the population) and ED50 (the amount of compound effective in 50% of the population). Therapeutic index data obtained from animal studies can be used in formulating a range of dosages for use in humans. The dosage of such compounds preferably lies within a range of plasma concentrations that include the ED50 with little or no toxicity. The dosage may vary within this range depending upon the dosage form employed and the route of administration utilized. The exact formulation, route of administration and dosage can be chosen by the individual physician in view of the patient's condition.

### EXAMPLES

### Materials and Methods

**Extraction and Isolation of IA.** Boswellia carterii resin (20 gr., Pamir, Tel Aviv, Israel) was extracted with PE (PE) (3 times with 150 ml). Petroleum ether (PE) extract was washed with NaOH 5% solution (3 times with 200 ml). The resulting aqueous acid-containing fraction was then acidified with HCl 1M, washed with saturated NaCl and re-extracted with PE. It was then dried over MgSO₄. The non acid containing PE fraction was acidified with HCl (1M) and then washed with a saturated NaCl solution and dried over MgSO₄. After evaporation the residue was chromatographed on a silica column. Fractions were assayed for their activity on IκB degradation as described below. A fraction eluted with 3% diethyl-ether in PE, which contained IA, showed activity. Pure IA was obtained by chromatography on a semi preparative HPLC column (Spectra-physics applied bio systems 783 absorbance detector with a vydac C18 semi-preparative HPLC column - Valco). Acetonitrile (ACN) and water were used as mobile phase for HPLC and the gradient consisted of 90-99% ACN for 30 min. A Waters HPLC instrument: pump 600, PDA 996 detector 600 with an analytical C18 Symmetry column (4.6/250 mm) were used to analyze the purification process. Several NMR methods (H-NMR, C-NMR, DEPT, COSY, HSQC, HMBC, TOCSY and NOESY) as well as a GC-MS analysis were used for the structure elucidation of the isolated active compounds.

NMR spectra were recorded both in CDC13 and in C6D6 solutions using a Bruker avance spectrometer 400 MHz and repeated using a Varian Unity Spectrometer Varian Unity Inova spectrometer 500 MHz.

GC-MS Analysis was performed using a Hewlett-Packard G1800A GCD system with a HP5971 gas chomatograph with an electron ionization detector. An SPB-5 (30 m x 0.25 mm x 0.25µm film thickness) column was used. The following method was used for analysis: The column was held at 70°C for 4 mins, after which, a temperature gradient was applied from 70°C to 280°C, at a rate of 50 degree/min. (Inlet temperature: 280°C; Detector temperature: 280°C; Splitless injection; gas - Helium, 1 mL/min).

**Cell Cultures.** HeLa cells were grown in Dulbecco's modified Eagle medium supplemented with 10% foetal calf serum and 1% (v/v) penicillin/streptomycin (all from Biological Industries, Kibbutz Beit Haemek, Israel), in a humidified incubator at 37° C.

RAW 264.7 macrophage cell line derived from BALB/c mice was obtained from American Type Culture collection (Rockville, MD, USA). The cells were cultured in Dulbecco's modified Eagle medium (DMEM) supplemented with 10% fetal calf serum (Hyclone, Logan, UT), 1% (v/v) penicillin/streptomycin (Beit Haemek, Israel), nonessential amino acid (Sigma, St. Louis, USA), glutamine 1% (Beit Haemek, Israel) and pyruvate 1% (Beit Haemek, Israel). Cells were grown in a humidified incubator at 37°C.

Peritoneal macrophages were harvested from C57BL/6 female mice four days after intraperitoneal injection of 1.5 ml of a 3% thioglycollate medium (Difco, NJ, USA). The cells were re-suspended in Dulbecco's modified Eagle medium (DMEM) supplemented with 5% foetal calf serum (FCS), and plated (1.2x10⁵ cells per well) in 96-microwell plates flat-bottom (Nunc, Roskide, Denmark).

**IκBα Degradation.** HeLa cells were pre-incubated with IA (50 µg/ml, dissolved in ethanol) for 2 hrs, and then stimulated for 20 minutes with TNFα (20 ng/ml, Emeryville, CA, USA). After removing the slides from plates for immonostaining (see below), proteins were extracted from remaining cells in the plates. Proteins were extracted from cells in NP-40 lysis buffer. Total protein concentration was determined using the Bradford method. Lysates were then analyzed either by Western blotting (WB).

**Western Blot (WB).** Following separation by SDS-PAGE, proteins were blotted to a polyvinylidene difluoride (PVDF) membrane (Millipore). The membrane was blocked in 5% (w/v) milk powder and then incubated in TBST containing the primary antibody and 2% (w/v) milk powder. All phospho-specific antibodies were purchased from Cell signaling Inc. αIκBα, αp65 and αCOX2 antibodies from Santa Cruz Inc. (California, USA). After binding of an appropriate secondary antibody coupled to horseradish peroxidase, proteins were visualized by enhanced chemiluminiscence according to the instructions of the manufacturer (Amersham Lifescience).

**p65 Subunit Immunostaining.** HeLa Cells were preincubated with IA and then stimulated with TNFα as described in the IκBα degradation assay above. Cells were then fixed with formaldehyde 1%, permeabilized with 0.25% Triton X-100, stained with rabbit anti-p65 (Santa Cruz, California, USA) and visualized with anti-rabbit Rhodamine Red-labeled secondary antibody (Jackson ImmunoResearch, Baltimore, USA). Cells were also stained with DAPI (blue) for nuclei location (data not shown). The cells were examined under an Axioscope Zeiss microscope with a plan-Neofluor * 60 lens.

**COX-2 Production.** RAW 264.7 cells were treated with subtoxic concentrations (confirmed by MTT colorimetric assay) of incensole acetate (10-20 µg/ml, dissolved in ethanol and further diluted in medium) and incubated with lipopolysaccharide (LPS, E. coli 1 µg/ml for 24 hs, Sigma, Israel) for 16-24 hrs. Cells treated with vehicle served as control group.

Proteins were extracted from cells in NP-40 lysis buffer (50 mM Tris/HCl pH 7.5, 150 mM NaCl, 0.1% SDS, 1% NP-40, 10 mM EDTA, 1 mM phenylmethylsulfonylfluoride (PMSF), and 10 mM DTT). Total protein concentration was determined using the Bradford method and the lysates were analyzed by Western blotting.

**Nitric Oxide (NO) Levels.** Following 2-3 h of incubation of murine peritoneal macrophages at 37°C, the non-adherent cells were removed by intensive rinsing. About 95% of the adherent cells were macrophages. IA was first dissolved in absolute ethanol, and the solutions were further diluted with Dulbecco's Modified Eagle's Medium (DMEM medium). Various nontoxic concentrations were added to the macrophages, followed by addition of 1 µg/ml of LPS for activation. The macrophages were then cultivated in a humid atmosphere with 5% CO₂ for 24 hrs. The supernatant fluids were harvested and kept at -20°C until assayed. NO generation was determined by measuring the nitrite accumulated in the supernatants (100 µl) of the IA-treated macrophages as follows. The cells were then treated with IA in various doses. An equal volume (100 µl) of Griess reagent (1% sulphanilamide, 0.1% naphthalene diamine HCl, 2% H₃PO₄) was added to each supernatant. Following 10 min of incubation at room temperature, the color production was measured at 550 nm with an ELISA reader. The concentration of the nitrite was calculated according to a standard curve.

**ROS (Reactive Oxygen Species) Production by RAW 264.7 Macrophages.** Raw 264.7 cells were scrapped, washed and resuspended in Hanks' balanced salt solution (without phenol red). For measurement of chemiluminescence, 0.5 ml of cell suspension (5 x 105 cells) was added to each luminometer tube, together with various doses of IA tested (dissolved in ethanol and further diluted with Hanks). The cells were incubated for 24 hrs. 10 µl of luminol (Sigma, St. Louis, USA) and 30 µl of zymosan (Sigma, St. Louis, USA) were added to the tubes, and the chemiluminescence was measured immediately in a luminometer (Biolumate LB 95, Berhold, Wilbad, Germany).

**Inflamed Paw Model.** Sabra female mice were used to assess the response to IA or vehicle in an *in vivo* model of inflammation. Drug or vehicle was administered 30 min before induction of the inflammatory stimulus. Mice (5 per group) were injected *i.p.* with vehicle (isopropanol:Emulphor:saline = 1:1:18) or with vehicle containing IA (50 mg/kg, *i.p*). Emulphor (a polyethoxylated vegetable oil) is a commercial emulsifier. Hind paws were injected with 50 µl of saline (left or right alternatively) or λ-carrageenin (4%, right or left alternatively), using 26G needles. Ensuing inflammatory swelling was measured by increase in foot volume in a plethysmometer (Ugo-Basile, Italy). Paw volume as well as redness (as a measure of erythema) and licking (as a measure of pain) were assayed before carrageenin application and every 60 min until 4 hrs.

**Statistical Analysis.** Student's t test was used to assess the differences between the control and IA-treated groups. For a dose response effect, analysis of the data was performed using a one way ANOVA followed by Bonferroni post-hoc comparisons. The paw model results were analyzed by ANOVA followed by Bonferroni post-hoc comparisons at every time point.

For analysis of c-Fos immunoreactivity, positive nuclei were identified based on their round form and optical density at least twice that of background. The numbers of c-Fos immunoreactive nuclei from the right and left hemispheres were averaged to obtain a representative number for the given region from each mouse. Student t tests were performed comparing the control (vehicle) with the IA group.

Responses to IA in WT versus TRPV3^{-/-}mice were assessed using two-way analysis-of-variance (ANOVA) with Bonferroni post-hoc comparisons (Graphpad Prism 4 software).

### Animals and Procedures

Female Sabra mice (Harlan, Israel, 2.5-3.5 months old) were used for the paw inflammatory model.. Female Sabra mice (Harlan, Israel, 15-20 weeks old) and wild type C57BL/6 or TRPV3 KO female mice (18-20 weeks old) were used for behavioral assessments. Ten mice were housed in each cage. For the chronic studies, mice were housed in groups of eight. Temperature in the animal room was maintained between 20-22°C, the light cycle was 12 h lights on (8-20h); 12 h lights off (20-8.00h). Female mice were used for all behavioural assessments, in order to prevent confounding due to potential wound infliction induced by inter-male fighting (See also below *"Animals and Procedures"* Section relating to Example 15.

Mice were consecutively tested in the elevated plus maze and the forced swimming test. The animal care and the protocols met the guidelines of the U.S. National Institutes of Health, detailed in the Guide for the Care and Use of Laboratory Animals, and were applied in conformity with the Institutional Ethics Committee.

### Drugs and Injections for Behavioral Assays

IA, IN and the extract were dissolved in a mixture of isopropanol: cremophor:saline=1:1:18. Injection volume was 10µl/g body weight. Injections were performed by the intraperitoneal (i.p.) route.

### Behavioral Assays

### Elevated Plus Maze

Mice were placed in the central platform (10 X 10 cm) between the open (10 X 45 cm) and enclosed (10 X 45 X 40 cm) arms of a plus maze. The number of entries and the time spent in each of the arms was recorded. As described by others (Crawley, 2000; Treit and Menard, 1998), an 'anti-anxiety' effect was calculated both as the ratio of entries onto the open arms to total arm entries, and as the % time on the open arms proportional to the time in the closed arms. Mice (female Sabra strain, aged 3.5-4.5 months old) were injected intraperitoneally with 10, 30 or 50 mg/kg of incensole acetate or with vehicle. Each dose was administered to 5 mice. Fifty min after injection the mice were tested in the plus-maze for 'anti-depressant' effects. Desipramine (5 mg/kg) was injected to a separate group of mice as a positive control. One-way Anova indicated significant effects (F = 8.9, df=4,27, P<0.01). All doses had 'anti-depressant' effects, but only those of 0 and 30 mg/kg were significant. Data are presented as means ± SEM. DMI=desipramine
*) P<0.05, **) P<0.01, ***) P<0.001 compared to vehicle

### Posrsolt's Forced swimming test (FST)

Mice were placed in a 2 liter glass beaker (11 cm diameter) filled with water (24±1°C) up to 30 cm from the bottom (so that the mouse could not touch the bottom and 8 cm from the rim (so that the mouse cannot escape). Immobility time (when the animal does not move except for small movements required to float) was recorded by 3 experimenters after 2, 6 and 9 min.

### For Example 15, the following experiments were conducted:

**Drug.** IA was isolated as described above under Materials and Methods. It was then dissolved in ethanol for in vitro assays or in isopropanol for in vivo assays. A stock solution of 20 mg/ml for in-vitro assays and 50 mg/ml for in vivo assay was prepared.

### Cell culture

Human HEK 293 cells stably expressing TRPV1 were a kind gift from Merck Research Laboratories (Whitehouse Station, NJ). Cells were cultured in minimal essential medium, Eagle, modified with non-essential amino acids, 1mM sodium pyruvate, 2 mM L-glutamine and 1.5 g/L sodium bicarbonate (ATCC, Mabassas, VA), containing 1% Penicillin-streptomycin, and 10% foetal bovine serum. Cells were passaged three times a week using Trypsin- EDTA 1 x (Invitrogen, Carlsbad, CA) and grown under 5% CO2 at 37°C.

TRPV3-YFP [O'dell, D.K., Rimmerman, N., Pickens, S.R. & Walker J.M. Fatty acyl amides of endogenous tetrahydroisoquinolines are active at the recombinant human TRPV1 receptor. Bioorg. Med. Chem. 15, 6164-6169 (2007)], TRPV4 and mock-transfected cell lines were cultured in DMEM 1x with L-glutamine (Mediatech, Inc. Herndon, VA), containing 1% penicillin-streptomycin (Invitrogen, Carlsbad, CA) and 10% foetal bovine serum.

HEK293 cells were transiently transfected with a rat TRPV2 plasmid using lipofectamine reagent (Invitrogen, Carlsbad, California) according to manufacturer's protocol. They were then maintained in Dulbecco's modified Eagle Medium / 10% fetal calf serum ,supplemented with Penicillin, Streptomycin, and L-glutamine. Primary keratinocytes from TRPV3-deficient and TRPV3+/+ mouse pups (day 1-4) were harvested and cultured as described previously [Chung, M.K., Lee, H., Mizuno, A., Suzuki, M. & Caterina, M.J. TRPV3 and TRPV4 mediate warmth-evoked currents in primary mouse keratinocytes. J. Biol. Chem. 279, 21569-21575 (2004)].

**Calcium imaging of HEK 293 cells.** TRPV1, TRPV3, and TRPV4 expressing HEK293 cells were plated 24-48 h before imaging in 96 well plates, loaded with 3 µM Fura-2 AM and imaged as previously described [O'dell, D.K., Rimmerman, N., Pickens, S.R. & Walker J.M. Fatty acyl amides of endogenous tetrahydroisoquinolines are active at the recombinant human TRPV1 receptor. Bioorg. Med. Chem. 15, 6164-6169 (2007)].

For single cell calcium imaging, HEK293-rat TRPV2 and HEK293-mouse TRPV3-YFP expressing cells were plated on collagen-coated glass cover slips. Cells were loaded for 60 min with 3 µM Fura-2 AM.

**Calcium imaging of TRPV3 +/+ and TRPV3 keratinocytes.** Primary keratinocytes from TRPV3-deficient and WT mouse pups (day 1-4) were harvested and cultured as described [Chung, M.K., Lee, H., Mizuno, A., Suzuki, M. & Caterina, M.J. TRPV3 and TRPV4 mediate warmth-evoked currents in primary mouse keratinocytes. J. Biol Chem. 279, 21569-21575 (2004)].

Cells were plated on glass coverslips (10⁵/cm²) and incubated for 48-60 h, then loaded with fura-2 AM (20 µM, 0.04% pleuronic acid, 32°C for 1h) in imaging buffer containing (in mM): 130 NaCl, 2.5 CaCl₂, 0.6 MgCl₂, 10 HEPES, 1.2 NaHCO₃, 10 glucose, pH 7.45. Ratiometric Ca²⁺ imaging was performed as previously described [O'dell, D.K., Rimmerman, N., Pickens, S.R. & Walker J.M. Fatty acyl amides of endogenous tetrahydroisoquinolines are active at the recombinant human TRPV1 receptor. Bioorg. Med. Chem. 15, 6164-6169 (2007)]. Drug was added to the bath following a period of baseline recording. Calcium measurements were made from 30 randomly selected cells per coverslip.

**Electrophysiological recording.** Currents were recorded using whole-cell voltage-clamp. Pipettes were pulled from microcapillary glass (A-M Systems). A coverslip containing cells was transferred to a 300 µL chamber that was constantly perfused (1-2 mL/min) with external solution. Voltage protocols were generated and data were digitized and recorded using Pulse (HEKA Elektronik) software in conjunction with an Axopatch 200A amplifier (Axon Instruments), and the data analyzed using an in-house Visual Basic (Microsoft) analysis program.

The pipette solution contained (in mM): 121.5 Kgluconate, 10 HEPES, 17 KCl, 9 NaCl, 1 MgCl₂, 0.2 EGTA, 2 MgATP, and 0.5 NaATP, pH 7.2. The external solution contained (in mM): 120 NaCl, 5 KCl, 1 MgCl₂, 2 CaCl₂, 10 Glucose and 20 HEPES, pH 7.4 with NaOH. The measured charge (pC) was defined as the charge elicited between-85 and -45 mV by a ramping voltage stimulus (-85 mV to +35 mV, 0.54 mV/msec; holding potential -55 mV). Currents were sampled at 5 kHz. Experimental and control cells were alternated whenever possible. Control values were obtained from adjacent cells with no detectable YFP fluorescence, presumed to be non-TRPV3-expressing.

**Data analysis of calcium imaging data.** Analysis of calcium imaging data was done using a non-linear regression curve fit (Graphpad 4 Prism, San Diego, CA). *t* tests and one way ANOVA were calculated using SPSS (Chicago, IL). In the keratinocyte experiments, drug-induced response for each cell was taken as the maximal post-drug measurements over time minus the average of the last 5 pre-drug measurements. Averaged drug responses over 30 randomly selected cells per coverslip were analyzed with two-tailed unpaired *t* tests.

For analysis of c-Fos immunoreactivity, positive nuclei were identified based on their round form and optical density at least twice that of background. The numbers of c-Fos immunoreactive nuclei from the right and left hemispheres were averaged to obtain a representative number for the given region from each mouse. Student *t* tests were performed comparing the control (vehicle) with the IA group.

Responses to IA in WT versus TRPV3^{-/-} mice were assessed using two-way analysis-of-variance (ANOVA) with Bonferroni post-hoc comparisons (Graphpad Prism 4 software).

**Animals and procedures.** Female Sabra mice (Harlan, Israel, 15-20 weeks old) and wild type C57BL/6 or TRPV3 KO female mice (18-20 weeks old) [Chung, M.K., Lee, H., Mizuno, A., Suzuki, M. & Caterina, M.J. TRPV3 and TRPV4 mediate warmth-evoked currents in primary mouse keratinocytes. J. Biol. Chem. 279, 21569-21575 (2004)] were used for behavioral assessments. 10 mice were housed in each cage. The animal care and protocols met the guidelines of the U.S. National Institutes of Health, detailed in the *Guide for the Care and Use of Laboratory Animals,* and were applied in conformity with the Institutional Ethics Committees. For the c-Fos immunostaining, female Sabra mice (see above) were used. Temperature in the animal room was maintained between 20-22°C, the light cycle was 12 h lights on (8:00-20:00h); 12 h lights off (20:00-8:00h). Mice were injected with intraperitoneal *(i.p.)* incensole acetate in a mixture of isopropanol:cremophor:saline (1:1:18) at a volume of 10 µl/g body weight

### Example 1: IA and IN Inhibit IκBα Degradation.

IA and IN were assayed at different concentrations for their activity on IκBα degradation in TNFα-stimulated HeLa cells. Both compounds inhibited IκBα degradation in a dose dependent manner **(****Fig. 1A****, 1B).**

### Example 2: IA Inhibits IκBα by Impairment of IKK Activity.

In order to demonstrate that IA inhibits the NF-κB pathway upstream from the IKKs experimentally, the effects of IA on TNFα-induced phosphorylation of the IKKs were tested. These experiments showed inhibition of IKKα/IKKβ phosphorylation by IA **(****Fig. 2A****).** Following IκBα degradation, NF-κB is free to accumulate in the nucleus. Immunostaining of the p65 sub-unit of NF-κB showed that IA inhibited the nuclear accumulation of NF-κB following TNFα stimulation in HeLa cells **(****Fig. 2B****).**

**Example 3: *IA Blocks NF-κB-mediated Inflammatory Response in vitro and in vivo.*** To investigate whether the NF-κB inhibitory effect of IA confers an anti-inflammatory activity, it was determined, as detailed herein above in Materials and Methods, the levels of COX-2, nitric oxide production and ROS with and without IA in different cell lines. The *in vivo* anti-inflammatory activity of IA was examined in inflamed paw model in mice. COX-2 production in LPS-stimulated RAW 264.7 cells was inhibited by IA at a dose of 60 µM (P<0.001) **(Fig. 3A).** NO production by murine peritoneal macrophages was determined by measuring the nitrite accumulated in the supernatants in an ELISA reader. IA inhibited NO generation in a dose dependent manner (ANOVA P<0.0001), reaching about 45% of NO production at 80 µM (p=0.0022) **(****Fig. 3B****).** ROS are known to be important in various biological and pathological processes and are involved in inflammation. We therefore tested the effects of IA on ROS generation by Zymozan activated Raw 264.7 cells at three concentrations. A significant dose-dependent inhibitory effect was found (ANOVA P<0.0001), reaching about 45% inhibition at 60 µM (p=0.0021) **(****Fig. 3C****).**

Having established that IA inhibits the expression of several key inflammatory mediators *in vitro,* the anti-inflammatory properties of IA *in vivo* were studies. It was thereupon found that IA significantly reduced inflammation in the inflamed paw model in mice during a 4 hrs period. The decreased inflamed paw volume in the treated mice reflects a decrease in edema, which is a component of the inflammatory response. There were highly significant effects of treatment (F=11.7, df=3,64, P<0.001), time (F=10.6, do-4,64, P<0.0001) and interaction (F=3.9, df=12,64, P<0.001) **(****Fig. 4****).** IA also significantly reduced other inflammatory parameters, such as redness and pain (data not shown).

### Example 5: Effect of IA on Post-CHI functional Outcome

To examine the effect of IA on functional recovery after CHI, the parameters of injured mice, treated with IA were compared with those of injured mice treated with vehicle.

At 1 h after CHI, the functional status of the mice was evaluated according to a set of 10 neurobehavioral tasks (neurological severity score, NSS) that tests reflexes, alertness coordination, and motor abilities. One point was awarded for absence of reflex or failure to perform a particular task. Hence, a score of 10 reflects maximal neurological impairment. Mice were equally divided to vehicle \ IA groups according to their NSS scores. Only mice with NSS > 4 at 1 h after injury were included in the study. Immediately after NSS1h assessment, mice were randomly assigned to intraperitoneal (i.p.) injection with vehicle (isopropanol: Emulphor - a commercial emulsifier: saline = 1:1:18) or with vehicle containing IA (50 mg/kg, n=9-10 mice/group). Recovery (ΔNSSt) was defined as the difference between NSS1h and NSS measured at any later time point and was determined at several time points up to 21 days following CHI.

NSS at 1h were similar in both groups, (7.03 ± 0.19 and 7.03 ± 0.19, respectively) indicating no difference in the initial severity of injury. Markedly greater recovery of motor ability was observed in the IA group 24 h after injury as compared with vehicle (ΔNSS = 1.00 ± 0.12 vs 0.41 ± 0.09 , respectively, P=0.002) as depicted in **Fig. 5A****.** ΔNSS values increased with time in both IA and vehicle mice as a result of spontaneous recovery, but continued to be significantly higher in IA mice at all subsequent time points, up to 3 weeks post injury.

### Example 6: Effect of IA on memory function

Memory function was assessed by ORT (Object Recognition Test) and the results are depicted in **Fig. 5****B.** Whereas naive, non-injured mice were not affected by IA (data not shown), it had a robust effect on the injured animals. Both groups spent equal time at the two objects (~50% of total exploration time) at the baseline measurements, at all times post CHI. However, at the test performed 4 h later, when a novel object replaced one of the familiar ones, IA-treated mice spent most of their exploration time at the new object, in contrast to the vehicle-treated animals, that did not memorize the "old" object. At 3 days post injury IA treated mice spent significantly longer times exploring the new object (P=0.01), similar to the time spent by a naive animal. This effect of IA was sustained for 7 and 14 days. At 21 days, it appears that the vehicle-treated mice regained their ability, and exploration time reached a similar level to that of the IA-treated mice.

### Example 7: Effect on tissue edema formation

A pronounced increase in tissue water content was observed in the left (ipsilateral) hemisphere of all injured mice at 24 h after injury, indicating the effect of injury in both groups. Although water accumulation tended to be smaller in IA mice (81.47 ± 0.35 % in IA vs 82.16 ± 0.30 % in vehicle) the difference did not reach statistical significance (P=0.15).

### Example 8: Effect of IA on cytokines expression profile after CHI

Since it was shown hereinabove that the pro-inflammatory cytokines TNF-α and IL-1β are upregulated within 1-4 h post-CHI, and that their inhibition is associated with better recovery. The mRNA levels of these cytokines were quantified at 3 hours after CHI using real-time PCR. Their amounts are expressed relative to β-actin, and it is apparent from **Fig. 6** that IA significantly inhibited mRNA expression of both TNF-α and IL-1β (P < 0.05, n=5/group).

### Example 9: Effect of IA on body temperature

Thirty minutes after treatment with IA (namely, 90 min post CHI), a mild (~1°C) and short-term (~30-60 min) duration of hypothermia was noted in IA- as compared to vehicle- treated mice (data is not shown).

### Example 10: The anxiolytic effect of IA:

When placed in an elevated plus-maze for the first time, a mouse's behavior is largely based on its anxiety level. Normal mice that have not received any anti-anxiety drugs will become moderately anxious in this new environment. Thus, they tend to prefer the closed arms over the less secure open arms. Meanwhile, mice treated with anti-anxiety drugs (e.g., diazepam, commonly known as valium) tend to be less anxious, so they spend more time in the 5 open arms compared to normal mice and they are generally less active. Forty five min after injection the mice were tested in the plus-maze for 'anti-anxiety' effects of IA **(****Fig. 7****).** Diazepam (5 mg/kg) was injected to a separate group of mice as a positive control. One-way Anova indicated significant effects (F = 4.2, df=4,32, P<0.01). Data are presented as means ± SEM.
*, P<0.05; **, P<0.01 compared to vehicle.

**Example 11:** The c-Fos transcription factor is a product of an immediate early gene and its increase serves as a marker of enhanced neuronal activity. It is thus used in histological sections to map out brain regions that are activated or attenuated after treatment with psychoactive drugs. IA significantly increased c-Fos in the lateral septum, central nucleus of the amygdala and solitary nucleus, while significantly reducing c-Fos in the motor cortex, medial striatum and hippocampal CA3 region **(****Fig. 9A****-C****).** The central nucleus of the amygdala and the lateral septum play major roles in **t**he expression of emotions; it is assumed that c-Fos expression in the central nucleus of the amygdala is due to circuits that are engaged by both anxiolytic and anxiogenic drugs.

The data from the behavioral assays together with the c-Fos immunostaining establish the anxiolytic and anti-depressive effects of IA.

**Example 12:** IA (75 mg/kg) exerted a potent anxiolytic-like effect in WT mice, while TRPV3^{-/-} mice spent identical time on the open arms, regardless of whether they were injected IA or only vehicle (**Fig. 10a****;** Fₛₜᵣₐᵢₙ = 6.3, df 1, 14, *p* < 0.05; F_{interaction} = 5.0, df 1,14, *p* < 0.05). In the Porsolt forced swim test, IA significantly reduced the immobility time in WT, but not in TRPV3^{-/-} mice (F_{IA} = 5.5, df 1,16, *p* < 0.04; F_{interaction} = 5.9, df 1,16, *p* < 0.03) **(****Fig. 10b****).** No significant differences were recorded between vehicle-treated WT mice and vehicle-treated TRPV3^{-/-} mice in the forced swim and elevated plus maze assays.

These results indicate that the effects of IA in preclinical models for antidepressants and anxiolytics are mediated via TRPV3 channels.

### Example 13: The anti-depressant effect of IA:

We used the Porsolt forced swimming test to examine the anti-depressant effect of IA. The method is based on the observation that a mouse, when forced to swim in a situation from which there is no escape, will, after an initial period of vigorous activity, eventually cease to move altogether making only those movements necessary to keep its head above water. This characteristic and readily identifiable behavioral immobility indicates a state of despair in which the rat has learned that escape is impossible and resigns itself to the experimental conditions. Fifty min after injection the mice were tested in the Porsolt forced swimming test for 'anti-depressant' effects **(****Fig. 8****).** Desipramine (5 mg/kg) was injected to a separate group of mice as a positive control. One-way Anova indicated significant effects (F = 8.9, df=4,27, P<0.01). Data are presented as means ± SEM. DMI=desipramine
*, P<0.05; * *, P<0.01; ***, P<0.001 compared to vehicle

### Example 15: IA effects on behavioral parameters

To study the functional effects of IA on the CNS, IA was assayed in a panel of standard behavioral assays in mice (female Sabra strain, 15-20 weeks old), namely: the elevated plus maze [Crawley, J.N. What's Wrong with my Mouse? Behavioral Phenotyping of Transgenic and Knockout Mice (Wiley-Liss, New York, 2000)], the Porsolt forced-swimming test [Petit-Demouliere, B., Chenu, F. & Bourin, M. Forced swimming test in mice: a review of antidepressant activity. Psychopharmacology 177, 245-255 (2005)], locomotion in the open field test and cataleptic response in a ring test [Fride, E. & Mechoulam, R. Pharmacological activity of the cannabinoid receptor agonist, anandamide, a brain constituent. Eur. J. Pharmacol. 231, 313-314 (1993). The elevated plus maze assay is based on the preference of mice for the closed arms of a maze, apparently due to fear of open spaces. At 50 mg/kg IA exerted a potent anxiolytic-like effect, causing mice to spend significantly more time in the aversive open arms of the maze. In the Porsolt forced-swim test, a standard assay for the evaluation of anti-depressant effects, IA significantly reduced the immobility recorded over 9 minutes, thus indicating a reversal of an avolition response. A significant ring. Dose dependency was noted in all assays (10-100 mg/kg, data not shown), and the findings were replicated in 7 independent experiments.

IA (100 µM) significantly increased calcium influx (EC₅₀= 16 µM; Hill slope = 2.2; **Fig. 12a****,b****,d**) in HEK293 cells stably expressing mouce TRPV3-YFP. When calcium was removed from the extracellular medium, the calcium increase in response to IA was significantly reduced **(****Fig. 12b****),** providing further evidence for the influx of calcium through TRPV3 channels. The effect of IA on TRPV3 resembles the effect of the broad-spectrum agonist 2-aminoethyl diphenylborinate (2-APB), which served as a positive control **(****Fig. 12a****,d****).** IA (500 µM) also induced a calcium influx in primary keratinocytes from WT mice, but not from TRPV3^{-/-} mice [Moqrich A., Hwang S.W., Earley T.J., Petrus M.J., Murray A.N., Spencer K.S., Andahazy M., Story G.M. & Patapoutian A. Impaired thermosensation in mice lacking TRPV3, a heat and camphor sensor in the skin. Science. 307, 1468-72 (2005)] **(****Fig. 12c****).** The effect of IA (500 µM) resembles the one of camphor (10 mM), a known agonist of TRPV3. IA, at a concentration (100 µM) that was maximally effective in TRPV3 expressing cells did not induce calcium influx in HEK293 cells transiently transfected with rat-TRPV2 **(****Fig. 12f****),** and caused only minimal calcium influx in HEK293 cells expressing rat TRPV and human TRPV4 **(****Fig. 12e****,g****).**

IA also activated a cation current in moue TRPV3-YFP expressing HEK293 cells **(****Fig. 13a****-c)** with properties consistent with TRPV3 activation [Smith G.D. et al. TRPV3 is a temperature-sensitive vanilloid receptor-like protein. Nature 418, 186-190 (2002)] and similar to the current activated by 2-APB, which served as a positive control **(****Fig. 13d****).** This current was not activated in HEK293 cells not expressing TRPV3 and was also absent from TRPV 1 and TRPV4 expressing cells **(****Fig. 13e****).**

The effect of IA on different brain regions were studied by looking at the effect of IA on c-Fos immunoreactivity in mice brains 60 min after administration of IA (50 mg/kg; *i.p.*). The c-Fos transcription factor is a product of an immediate early gene and its increase serves as a marker of enhanced neuronal activity. It is thus used in histological sections to map out brain regions that are activated or attenuated after treatment with psychoactive drugs [Werme, M., Ringholm, A., Olson, L. & Brene S. Differential patterns of induction of NGFI-B, Norl and c-fos mRNAs in striatal subregions by haloperidol and clozapine. Brain Res. 863, 112-119 (2000); and Dragunow, M., Robertson, G.S., Faull, R.L., Robertson, H.A. & Jansen, K. D2 dopamine receptor antagonists induce fos and related proteins in rat striatal neurons. (1990) Neuroscience 37, 287-294]. IA significantly increased c-Fos in the lateral septum, central nucleus of the amygdala and solitary nucleus, while significantly reducing c-Fos in the motor cortex, medial striatum and hippocampal CA3 region (**Fig. 9****;** Table 1). The central nucleus of the amygdala and the lateral septum play major roles in the expression of emotions [Thompson, B.L. & Rosen, J.B. Immediate-early gene expression in the central nucleus of the amygdala is not specific for anxiolytic or anxiogenic drugs. Neuropharmacology 50, 57-68 (2006); and Henry, B., Vale, W. & Markou, A. The effect of lateral septum corticotropin-releasing factor receptor 2 activation on anxiety is modulated by stress. J. Neurosci. 26, 9142-9152 (2006)]; it is assumed that c-Fos expression in the central nucleus of the amygdala is due to circuits that are engaged by both anxiolytic and anxiogenic drugs [Thompson, B.L. & Rosen, J.B. Immediate-early gene expression in the central nucleus of the amygdala is not specific for anxiolytic or anxiogenic drugs. Neuropharmacology 50, 57-68 (2006).]

The data from the behavioral assays together with the c-Fos immunostaining establish the anxiolytic and anti-depressive effects of IA. Given the robust effect of IA on TRPV3 channels and the observation that IA does not interact with a long list of receptors known to be involved in psychoactivity, the possibility that its behavioral effects are mediated through CNS TRPV3 channels was investigated. Thus, the panel of behavioral assays with WT and TRPV3^{-/-} mice, which were administered either IA or vehicle was repeated.

IA (75 mg/kg) exerted a potent anxiolytic-like effect in WT mice, while TRPV3^{-/-}mice spent identical time on the open arms, regardless of whether they were injected IA or only vehicle (**Fig. 10a****;** Fₛₜᵣₐᵢₙ= 6.3, df 1, *14, p* < 0.05; F_{interaction} = 5.0, df 1,14, *p* < 0.05). In the Porsolt forced swim test, IA significantly reduced the immobility time in WT, but not in TRPV3^{-/-} mice (F_{IA} = 5.5, df 1,16, *p* < 0.04; F_{interaction} = 5.9, df 1,16, *p* < 0.03) **(****Fig. 10b****).** No significant differences were recorded between vehicle-treated WT mice and vehicle-treated TRPV3^{-/-} mice in the forced swim and elevated plus maze assays.

These results indicate that the effects of IA in preclinical models for antidepressants and anxiolytics are mediated via TRPV3 channels.

Collectively, the data presented here, along with the expression of TRPV3 mRNA in the brain, indicate that TRPV3 channels affects emotional and behavioral processes in the CNS, in addition to its known effects on thermosensation.

### References:

The Complete Parallel Bible, Proverbs, 27, 9, (Oxford University Press, 1989).
Ammon, H.P., Safayhi H., Mack T. and Sabieraj J. (1993) J Ethnopharmacol 38,113-9.
Barnes P.J., Karin M. (1997) N. Engl J Med.336, 1066-71.
Beni SM, Kohen R, Reiter RJ, Tan DX, Shohami E (2004) Melatonin-induced neuroprotection after closed head injury is associated with increased brain antioxidants and attenuated late-phase activation of NF-kappaB and AP-1. FASEB J 18, 149-51.
Chung, M.K., Lee, H., Mizuno, A., Suzuki, M. & Caterina, M.J. TRPV3 and TRPV4 mediate warmth-evoked currents in primary mouse keratinocytes. J. Biol. Chem. 279, 21569-21575 (2004).
Crawley, J.N. What's Wrong with my Mouse? Behavioral Phenotyping, of Transgenic and Knockout Mice (Wiley-Liss, New York, 2000).
Corsano, S. & Nicoletti, R. The structure of incensole. Tetrahedron 23, 1977-1984 (1967).
Dragunow, M., Robertson, G.S., Faull, R.L., Robertson, H.A. & Jansen, K. D2 dopamine receptor antagonists induce fos and related proteins in rat striatal neurons. (1990) Neuroscience 37, 287-294.
Foo S.Y., Nolan G.P. Trends Genet. (1999) 15 229-35.
Fride, E. & Mechoulam, R. Pharmacological activity of the cannabinoid receptor agonist, anandamide, a brain constituent. Eur. J. Pharmacol. 231, 313-314 (1993).
Gacs-Baitz E., Radics L. Fardella G. And Corsano S. (1978) Journal of Chemical Research (S), 1701-1709.
Gerhardt, H, Seifert, F, Buvari, P, Vogelsang, H, Repges, R. (2001) Zeitschrift-fur-Gastroenterologie 39, 7-11.
Ghosh S. and Karin M. (2002) Cell 109, S81-S96.
Gupta, I., Gupta, V., Parihar, A., Gupta, S., Ludtke, R., Safayhi, H., Ammon, H.P. (1998) Eur J Med Res 3, 511-4.
Hansson G.K. (2001) Ann N Y Acad Sci. 947 157-65; discussion 165-6.
Haoxing Xu et al. Nature Neuroscience (2006) 9, 628-635.
Henry, B., Vale, W. & Markou, A. The effect of lateral septum corticotropin-releasing factor receptor 2 activation on anxiety is modulated by stress. J. Neurosci. 26, 9142-9152 (2006).
H. Z. Hu et al., J Biol Chem 279, 35741 (Aug 20, 2004).
Karin M. and Ben-Neriah Y. (2000) Annual Review of Immunology 18, 621-663.
T. Kato, C.C. Yen, T. Kobayashi, Y. Kitahara. Cyclization of polyenes XXI. Synthesis of DL-incensole. Chemistry letters 1191-1192 (1976),
H. Lee, M. J. Caterina, Pflugers Arch 451, 160 (Oct, 2005).
Marmarou A, Guy M, Murphey L, Roy F, Layani L, Combal JP, Marquer C; American Brain Injury Consortium (2005) A single dose, three-arm, placebo-controlled, phase I study of the bradykinin B2 receptor antagonist Anatibant (LF16-0687Ms) in patients with severe traumatic brain injury. J Neurotrauma 22, 1444-55.
Moqrich A., Hwang S.W., Earley T.J., Petrus M.J., Murray A.N., Spencer K.S., Andahazy M., Story G.M. & Patapoutian A. Impaired thermosensation in mice lacking TRPV3, a heat and camphor sensor in the skin. Science. 307, 1468-72 (2005).
Narayan RK, Michel ME, Ansell B, et al (2002) Clinical trials in head injury. J Neurotrauma 19, 503-57.
Nonaka M, Chen XH, Pierce JE, Leoni MJ, McIntosh TK, Wolf JA, Smith DH (1999) Prolonged activation of NF-kappaB following traumatic brain injury in rats. J Neurotrauma 16, 1023-34.
O'dell, D.K., Rimmerman, N., Pickens, S.R. & Walker J.M. Fatty acyl amides of endogenous tetrahydroisoquinolines are active at the recombinant human TRPV1 receptor. Bioorg. Med. Chem. 15, 6164-6169 (2007).
Paxinos G. & Franklin K.B.J. The Mouse Brain in Stereotaxic Coordinates (Academic Press, Oxford, 2001).
Petit-Demouliere, B., Chenu, F. & Bourin, M. Forced swimming test in mice: a review of antidepressant activity. Psychopharmacology 177, 245-255 (2005).
Singh G.B, Atal C.K. (1986) Agents and Actions 18, 407-412.
Smith G.D. et al., Nature 418, 186 (Jul 11, 2002).
Syrovets, T., Buchele, B., Krauss, C., Laumonnier, Y., and Simmet, T. (2005), J Immunol. 174, 498-506.
Strappaghetti, G., Proietti G., Proietti, S. Corsano, S. and I. Grgurina, I. Synthesis of incensole. Bioorganic Chemistry 11, 1-3 (1982).
Thompson, B.L. & Rosen, J.B. Immediate-early gene expression in the central nucleus of the amygdala is not specific for anxiolytic or anxiogenic drugs. Neuropharmacology 50, 57-68 (2006).
Treit, D. & Menard, J. in In vivo Neuromethods eds Boulton, A., Baker, G. & Bateson, A. (Humana Press, Totowa, 1998), p 89.
Vogt-Eisele, AK et al., Monoterpenoid agonists of TRPV3, British Journal of Pharmacology (2007) 151, 530-540. Watt, J.M. (1962) The Medicinal and Poisonous Plants of Southern and Eastern Africa, 2nd Ed., Livingstone LTD., Edinburgh and London.
Werme, M., Ringholm, A., Olson, L. & Brene S. Differential patterns of induction of NGFI-B, Nor1 and c-fos mRNAs in striatal subregions by haloperidol and clozapine. Brain Res. 863, 112-119 (2000).
US Patent No. 5064823
WO 02/053138

## Claims

1. A compound having the structural formula I, including enantiomers, diastereomers, solvates, and pharmaceutically acceptable salts thereof: 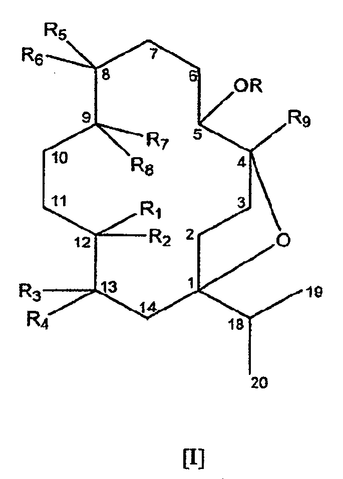 wherein,
R is selected from H, -C(=O)R', and -C(=O)OR", wherein R' is C₁₋₂alkyl and R" is H or C₁₋₂₅alkyl; **R₁, R₂, R₅,** and **R₆** are independently selected from H, OH and CH₃;
**R₃, R₄, R₇,** and **R₈** are independently selected H and OH;
**R₉** is H or CH₃; or
one of **R₁** and **R₂** and one of **R₃** and **R₄** taken together form (i) a second bond between C₁₂ and C₁₃ or (ii) an epoxide ring, along with the carbon to which they are bonded; and/or
one of **R₅** and **R₆** and one of **R₇** and **R₈ taken** together form (iii) a second bond between C₈ and C₉ or (iv) an epoxide ring, along with the carbon to which they are bonded; and/or
one of **R₅** and **R₆** together with R form a single bond, thereby forming an epoxide ring along with the carbons to which they are bonded,
for use in neuroprotection.

2. The compound for use according to claim 1, wherein said neuroprotection is for treatment, prevention or amelioration of a disease or condition resulting from injury, trauma or CNS neurodegenerative diseases.

3. A compound having the structural formula I as defined in claim 1 for use in the treatment, prevention or amelioration of a disease or condition selected from depression, anxiety, obsessive compulsive behaviors, deterioration in cognitive function, and deterioration in neurobehavioral function.

4. A TRPV3 agonist having the structural formula I as defined in claim 1 for use in the treatment of a disease or condition selected from mood-disorders, anxiety, and a combination thereof.

5. The compound for use according to any one of claims 1, 2, 3 or 4, wherein said compound is incensole or incensole acetate.

6. The compound for use according to claim 5, wherein said compound is incensole.

7. The compound for use according to claim 5, wherein said compound is incensole acetate.

## Patentansprüche

1. Eine Substanz mit der Strukturformel I einschließlich deren Enantiomere, Diasteromere, Lösungsprodukte und pharmazeutisch annehmbare Salze derselben: 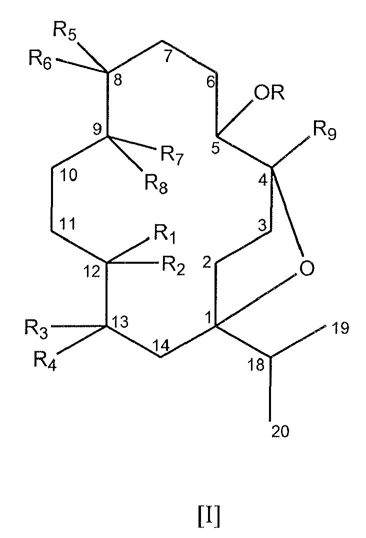 wobei,
**R** ausgewählt ist aus H, -C(=O)R', und -C(=O)OR", wobei R' ein C₁₋₂₅alkyl and R" ein H oder C₁₋₂₅alkyl sind; **R₁, R₂, R₅,** und **R₆** unabhängig voneinander ausgewählt sind von H, OH und CH₃;
**R₃, R₄, R₇,** und **R₈** unabhängig voneinander ausgewählt sind aus H und OH;
**R₉,** H oder CH₃ sind; oder
eine von **R₁** und **R₂** und eine von **R₃** and **R₄** zusammen (i) eine zweite Bindung zwischen C₁₂ und C₁₃ oder (ii) einen Epoxidring zusammen mit dem Kohlenstoff, an welches sie gebunden sind, bilden; und/oder
eine von **R₅** und **R₆** and eine von **R₇** und **R₈** zusammen (iii) eine zweite Bindung zwischen C₈ und C₉ oder (iv) einen Epoxidring, zusammen mit dem Kohlenstoff, an welches sie gebunden sind, bilden; und/oder
eine von **R₅** und **R₆** zusammen mit R. eine Einfachbindung eingeht, durch welche ein Epoxidring mit dem Kohlenstoff, an welches sie gebunden sind, bildet,
für die Verwendung der Neuroprotektion.

2. Die Substanz zur Verwendung gemäß Anspruch 1, wobei besagte Neuroprotektion die Behandlung, Vorbeugung und Verbesserung einer Krankheit oder Zuständen resultierend aus Verletzung, Trauma oder neurodegenerativen ZNS Erkrankungen einschließt.

3. Eine Substanz mit der Strukturformel I wie in Anspruch 1 definiert für die Verwendung zur Behandlung, Vorbeugung und Verbesserung einer Krankheit oder Zuständen ausgewählt aus Depression, Angst, Zwangsverhaltens, Verschlechterung der kognitiven Fähigkeiten und Verschlechterung in neuronalen Verhaltensfunktionen.

4. Ein TRPV3 Agonist, gemäß der Strukturformel I wie in Anspruch 1 definiert, zur Verwendung in der Therapie einer Krankheit oder eines Zustandes ausgewählt aus affektiven Störungen, Angstzuständen oder Kombinationen derselben.

5. Eine Substanz zur Verwendung gemäß einem der Ansprüche 1, 2, 3 oder 4 wobei besagte Substanz ein Incensol oder Incensolacetat ist.

6. Die Substanz zur Verwendung gemäß Anspruch 5 wobei, besagte Substanz Incensol ist.

7. Die Substanz zur Verwendung gemäß Anspruch 5, wobei besagte Substanz Incensolacetat ist.

## Revendications

1. Composant possédant la formule structurelle I, comportant des énantiomères, des diastéréomères, des solvates, et des sels pharmaceutiquement acceptables de ceux-ci : 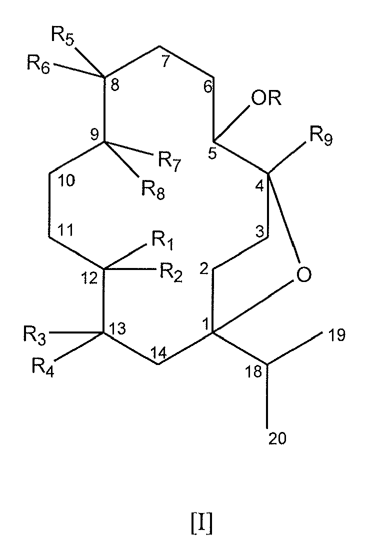 dans lequel
**R** est sélectionné à partir de H, -C(=O)R', et -C(=O)OR", dans lequel R' est de l'alkyle C₁₋₂₅ et R" est du H ou de l'alkyle C₁₋₂₅ ; **R₁, R₂, R₅,** et **R₆** sont sélectionnés indépendamment à partir du H, OH et CH₃ ;
**R₃, R₄, R₇,** et **R₈** sont indépendamment du H et du OH sélectionnés ;
**R₉** est du H ou du CH₃ ; ou
l'un de **R₁** et **R₂** et l'un de **R₃** et **R₄** pris ensemble forment (i) une seconde liaison entre C₁₂ et C₁₃ ou (ii) un composé cyclique d'époxyde, ensemble avec le carbone auquel ils sont reliés ; et/ou
l'un de **R₅** et **R₆** et l'un de **R₇** et **R₈** pris ensemble forment (iii) une seconde liaison entre C₈ et C₉ ou (iv) un composé cyclique d'époxyde, ensemble avec le carbone auquel ils sont reliés ; et/ou
l'un de **R₅** et **R₆** ensemble avec R forment une liaison unique, formant alors un composé cyclique d'époxyde, ensemble avec le carbone auquel ils sont reliés,
en vue de l'utilisation dans la neuroprotection.

2. Composant destiné à l'utilisation selon la revendication 1, dans lequel ladite neuroprotection est destinée au traitement, à la prévention ou l'amélioration d'une maladie ou d'une condition résultant d'une blessure, d'un traumatisme ou de maladies neurodégénératives du système nerveux central.

3. Composé possédant la formule structurelle 1 telle que définie dans la revendication 1, destiné à l'utilisation dans le traitement, la prévention ou l'amélioration d'une maladie ou d'une condition sélectionnées à partir de la dépression, de l'anxiété, des comportements obsessifs compulsifs, de la détérioration de la fonction cognitive, et de la détérioration de la fonction neurocomportementale.

4. Agoniste TRPV3 possédant la formule structurelle I telle que définie dans la revendication 1, destiné à l'utilisation dans le traitement d'une maladie ou d'une condition sélectionnées à partir des troubles de l'humeur, de l'anxiété ou d'une combinaison de celles-ci.

5. Composant destiné à l'utilisation selon une quelconque des revendications 1, 2, 3 ou 4, dans lequel ledit composant est de l'incensole ou de l'acétate d'incensole.

6. Composant destiné à l'utilisation selon la revendication 5, dans laquelle ledit composant est de l'incensole.

7. Composant destiné à l'utilisation selon la revendication 5, dans laquelle ledit composant est de l'acétate d'incensole.
